# EUROPEAN PATENT SPECIFICATION

(11) **EP 1 443 958 B1**
(45) Date of publication and mention of the grant of the patent: **08.12.2010**
(21) Application number: 02800662.5
(22) Date of filing: 07.10.2002
(51) Int. Cl.: A61K 39/00, C07K 14/435, C07K 16/18, A61P 31/00, A61P 31/04, A61P 31/12, A61P 25/00

(54) **VACCINE AGAINST INFECTIOUS DISEASE**
VAKZINE GEGEN INFEKTIONSKRANKHEITEN
VACCIN CONTRE DES MALADIES INFECTIEUSES

(30) Priority: 08.10.2001 GB 0124137; 20.03.2002 GB 0206575
(43) Date of publication of application: 11.08.2004
(73) Proprietor: Merial Limited, Harlow, Essex CM19 5QA (GB)
(72) Inventor: NUTTALL, P. A., Centre for Ecology & Hydrology, Oxford OX1 3SR (GB); TRIMNELL, A. R., Seattle WA 98109-5219 (US); LABUDA, Milan, 900 32 Borinka (SK); KAZIMIROVA, Maria, 900 42 Dunajska Luzna (SK); LICKOVA, Martina, 841 01 Bratislava (SK)
(74) Representative: Harding, Charles Thomas
(86) International application number: PCT/GB2002/004546
(87) International publication number: WO 2003/030931

(56) References cited:
- WO-A-01/40469
- WO-A-01/80881
- WO-A-99/24567
- WANG H ET AL: "IMMUNOGLOBULIN-BINDING PROTEINS IN TICKS: NEW TARGET FOR VACCINE DEVELOPMENT AGAINST A BLOOD-FEEDING PARASITE" CMLS CELLULAR AND MOLECULAR LIFE SCIENCES, BIRKHAUSER VERLAG, BASEL, CH, vol. 56, no. 3/4, 1999, pages 286-295, XP001009780 ISSN: 1420-682X

## Description

The present invention relates to the use of a protein termed 64p in the production of vaccines for protecting animals against the bite of blood-sucking ectoparasites and against the transmission of viruses, bacteria and other pathogens by such ectoparasites.

Blood-sucking ectoparasites, such as mosquitoes, horseflies, tsetse flies, fleas, lice, mites and ticks, are extremely effective as transmitters of disease. Mosquito borne diseases include Malaria (*Plasmodium* parasites transmitted by *Anopheles* mosquitoes), Dengue Fever, Yellow Fever and Arboviral Encephalitides (such as Eastern Equine Encephalitis, Japanese Encephalitis, La Crosse Encephalitis, St. Louis Encephalitis (*Culex pipiens* mosquitoes). Western Equine Encephalitis and West Nile Virus Encephalitis), Lymphatic filariasis (elephantiasis). Other diseases that are borne by blood-sucking ectoparasite vectors include plague (flea); Schistosomiasis (flatworms); trypanosomiasis (tsetse fly), Leishmaniasis (sandfly), and Onchocerciasis (blackfly).

Taking the tick as an example, these arthropods are able to transmit protozoan, rickettsial and viral diseases of livestock, which are of great economic importance world-wide. Losses to the livestock industry, in particular the production of cattle and small ruminants in tropical and sub-tropical areas, have been estimated to be in the range of several billion US dollars annually. In many developing countries, tick-borne protozoan diseases, including *Theileria parva* which causes the usually fatal East Coast Fever (Norval *et al.,* 1992a; Norval *et al*., 1992b), babesioses and rickettsial diseases such as anaplasmoses, cowdriosis and tick-associated dermatophilosis, are major health and management problems of livestock. Furthermore, tick pests also cause considerable damage to animals' skin, thereby affecting the leather industry. Ticks also act as transmitters of human disease, including Lyme disease (*Borrelia burgdorferi,* transmitted by *Ixodes scapularis*), Southern Tick-Associated Rash Illness (STARI), Babesiosis, Ehrlichiosis, Rocky Mountain Spotted Fever (caused by *Rickettsia rickettsia* transmitted by *Dermacentor variabilis*) and Crimean-Congo Haemorrhagic Fever.

Normally, these disease agents can only be transmitted by the bite of an infected ectoparasite. For example, ticks normally become infected by taking a blood meal from an infected animal. Male, female and immature (nymphs and larvae) ticks feed on blood and all stages are capable of transmitting disease agents. There are four stages in the life cycle of a tick: egg, larval, nymph, and adult. It generally takes several months to two years to complete this life cycle. A blood meal is taken in all except the egg stage. After each blood meal, the cuticle is shed and the tick matures to its next life stage. Thus it is possible for a tick to transmit disease organisms three times in its life. It is also possible to became infected by handing infected ticks, such as when removing ticks from a pet, when infective tick body fluids are introduced into a wound or mncus membrane.

In an effort to combat tick-transmitted diseases, a number of attempts have been made to immunise animals against ticks using extracts of whole ticks or of tick gat. Certain reports have used recombinant tick proteins (see, for example, International patent application WO88/03929). WO01/80881 reports the generation of vaccines that incorporate a protein termed 64P and fragments thereof. However, despite such developments, the only commercially-available tick vaccines are active only against the adult stage of a few tick species and show variation in efficacy depending on the geographical location of the species. No vaccines have yet been developed that provide resistance across entire populous of vaccinated animals or against parasites at every stage of their life cycle. WO01/80881 is a citation under article 54(3) EPC.

WO99/24567 discloses tissue cement proteins produced by certain species of blood-feeding ectoparasites.

There is a great need to reduce the incidence of infectious diseases that are caused by blood-feeding ectoparasites, particularly in tropical and sub-tropical regions, where such ectoparasites, and the disease agents carried by them, are endemic. A large number of strategies are being pursued in order to try and eradicate these diseases, and these mainly focus on attempting to limit the numbers of the ectoparasites themselves. However, to date, none of these strategies has yet shown any enduring success. Indeed, as the global climate warms, it is likely that areas not previously afflicted by infections diseases such as malaria will become vulnerable. The recent outbreak of human encephalitis cases in the north-eastern United Stares, caused by mosquito-borne West Nile virus, serves to illustrate the potential problems facing areas that are currently temperate over the coming years.

There therefore exists a great need for an effective vaccine to combat diseases that are transmitted by blood-feeding ectoparasites. It has now been discovered that a protein teamed 64p, originally isolated in the tick, is useful as a vaccine component.

### Summary of the invention

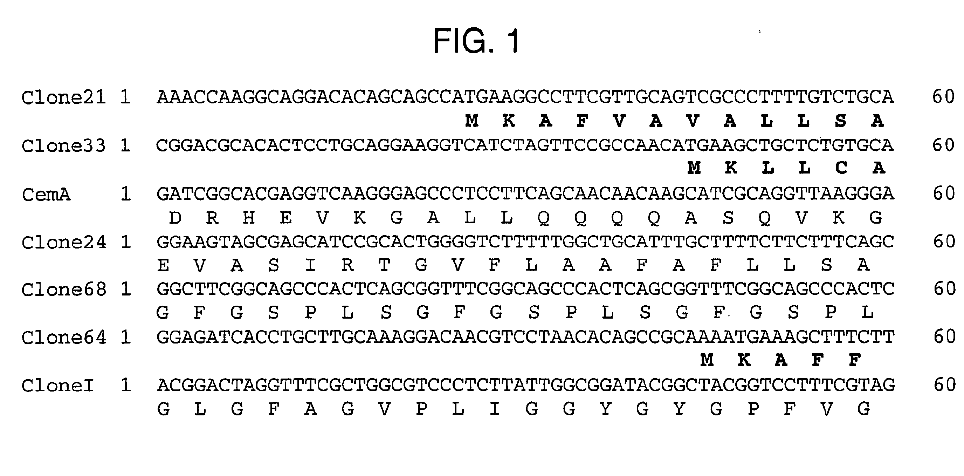

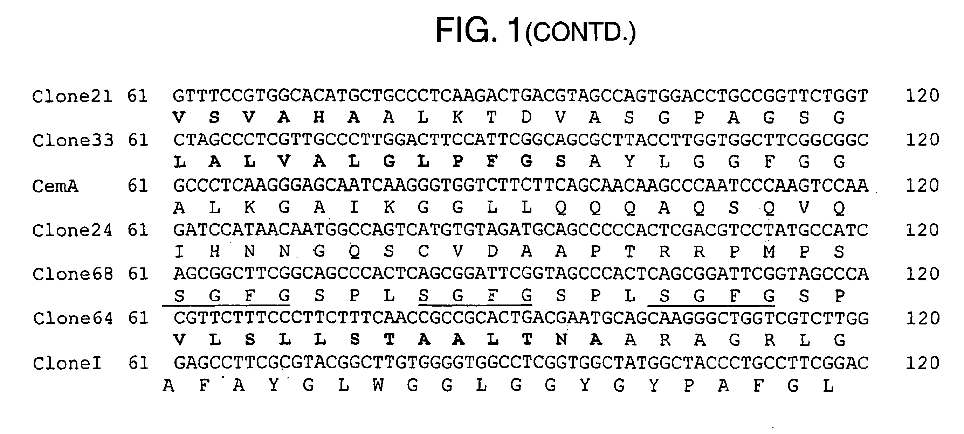

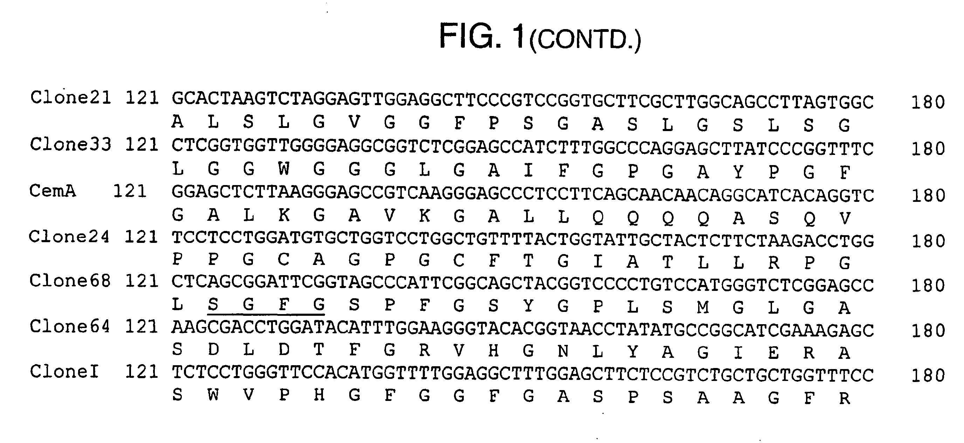

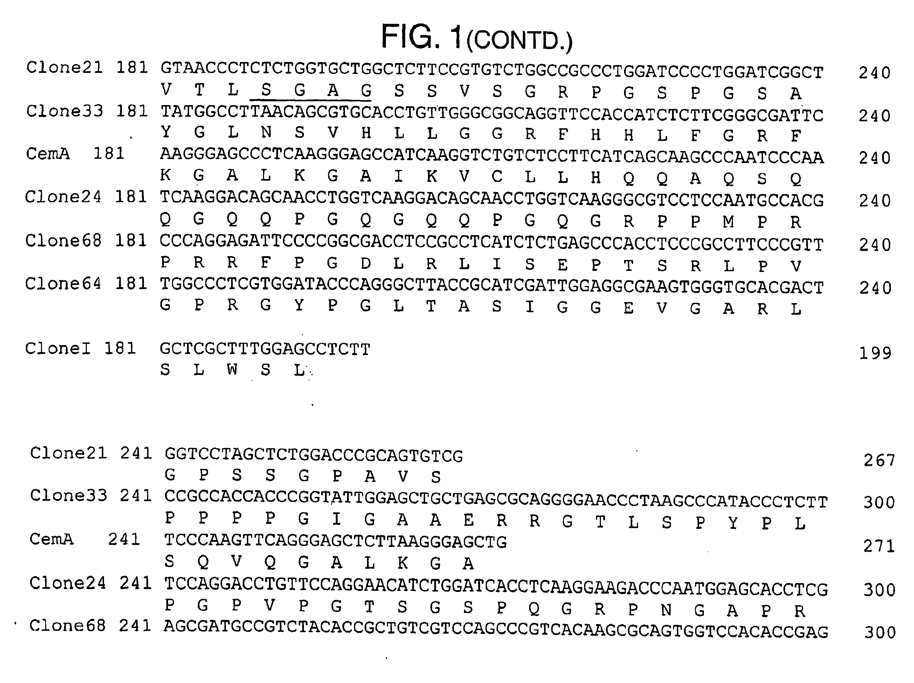

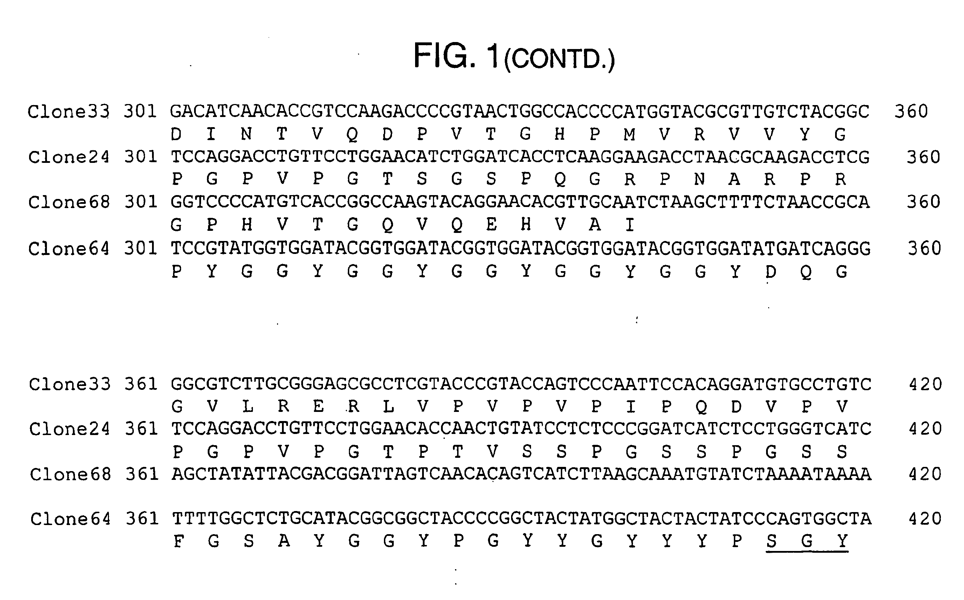

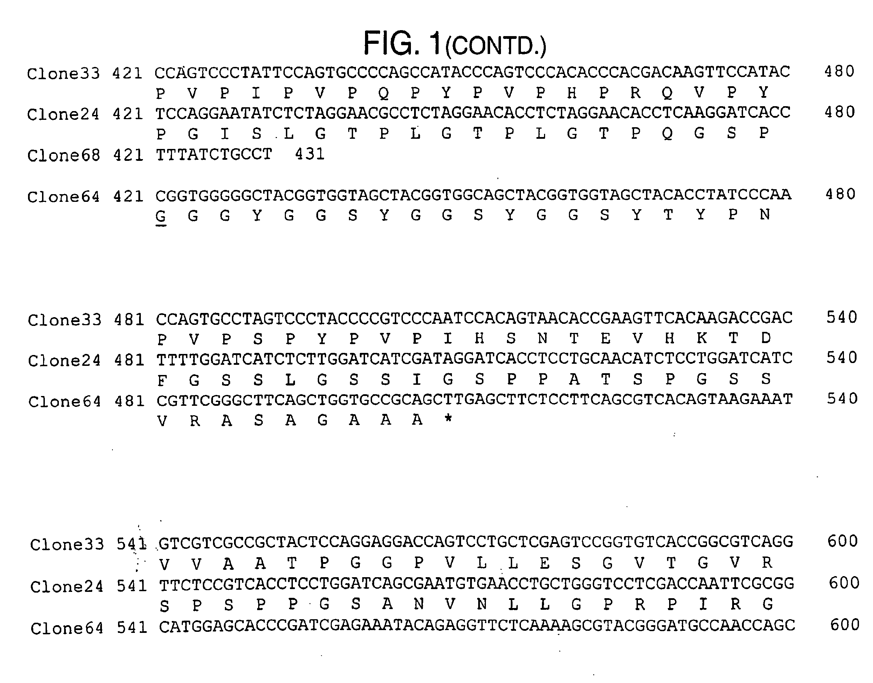

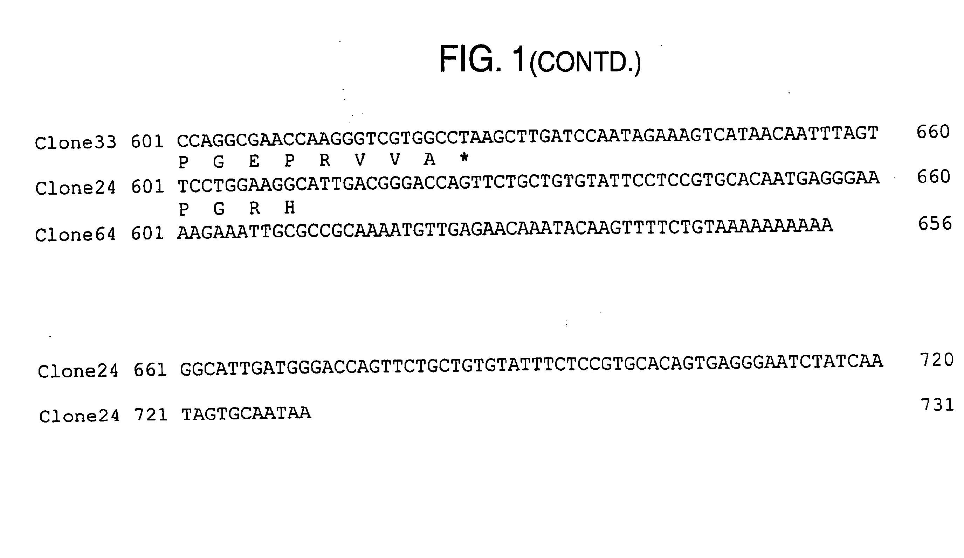

The present invention provides, in one aspect, the use of an immunogenic 64p protein consisting of the amino acid sequence presented in Figure 1, an immunogenic fragment thereof or an immunogenic homologue of said 64p protein or fragment having at least 50% identity thereto, or a plasmid DNA vector encoding said protein, fragment or homologue; for the preparation of a vaccine composition for the prevention of tick-borne encephalitis (TBE) and/or to reduce the transmission of the TBE virus to uninfected ticks; or for the preparation of a vaccine composition for the prevention of a mosquito-borne disease and/or reduction in transmission of a mosquito-borne disease; for the preparation of a vaccine composition for the prevention of tick-borne encephalitis (TBE) and/or to reduce the transmission of the TBE virus to uninfected ticks; wherein said vaccine composition is for administration to animals.

The present invention provides a vaccine composition comprising immunogenic 64p protein consisting of the amino acid sequence presented in Figure 1, an immunogenic fragment thereof or an immunogenic homologue of said 64p protein or fragment having at least 50% identity thereto, or a plasmid DNA vector encoding said protein, fragment or homologue; for use in the prevention of tick-borne encephalitis (TBE) and/or reduction in transmission of the TBE virus to uninfected ticks; or for use in the prevention of a mosquito-borne disease and/or reduction in transmission of a mosquito-borne disease; wherein said vaccine composition is for administration to animals.

There is provided herein a vaccine effective against the transmission of an infectious disease borne by an ectoparasite, said vaccine comprising as an active component a 64p protein consisting of the sequence presented in Figure 1, a fragment thereof or a homologue of said 64p protein or protein fragment that exhibits at least 50% sequence identity with said protein or protein fragment. Immunisation of an animal with such a vaccine is shown herein to cause the generation of antibodies that are effective against a wide variety of ectoparasite species. The vaccine is also shown to impart protection against the transmission of an infectious disease via a blood-sucking ectoparasite.

Although the Applicant does not want to be confined to any particular theory, it appears that the vaccines mentioned herein work in the following way. Immunisation of a host species with the 64p protein, fragment or homologue elicits an immune response against the ectoparasite protein. This stimulates an inflammatory response that boosts the immune status of vaccinated animals. However, additionally, the 64p protein and fragments thereof have been discovered to contain epitopes that also exist in proteins that are present in the salivary glands, gut and haemolymph of a large number of ectoparasite species. This cross-reactivity makes the vaccines mentioned herein particularly advantageous, since ingestion of blood, and thus host antibodies, into the ectoparasite guarantees delivery of the active agent to the parasite. In this manner, the vaccines mentioned herein target species that feed transitorily, such as mosquitoes and horseflies, as efficiently as those species that remain attached to their host for a significant period of time, such as ticks.

The nature of the immune response that the vaccines mentioned herein impart is responsible for the decreased transmission of the agent that causes the infectious disease borne by the ectoparasite. Because of the presence in the host of antibodies that recognise not only a protein species present in the ectoparasite saliva, but also a protein species that is found in the gut, the blood-feeding event, however transitory, is sufficient to allow the transmission to the ectoparasite of antibodies that lead to its death. Immunisation of host animals with a vaccine according to the description thus leads to a decrease in the actual numbers of ectoparasites, as well as a concomitant decrease in the numbers of ectoparasites carrying disease-causing agents. This has a significant effect on the incidence of disease *per se.*

A large number of ectoparasite species exist in various parts of the world, although their incidence tends to be concentrated in tropical and sub-tropical regions, where they, and diseases carried by them, are endemic. These species vary greatly in type and adopt widely differing feeding strategies, ranging from transient feeders such as mosquitoes, horseflies, sandflies, blackflies, tsetse flies, fleas, lice and mites, down to flatworms and ticks, some of which may feed for long periods of time. All of these ectoparasite species are suitable targets for the vaccines of the description.

What is common between all these ectoparasite species is that they ingest either blood, lymph or they feed on host skin products, meaning that any antibodies present in their host are automatically internalised into the ectoparasite. This provides an advantageous and automatic route of administration for antibody and, provided that the antibody is reactive against an ectoparasite protein, means that a well-organized immunisation regime can result in the complete eradication of the parasite within the area concerned. Ectoparasites that feed on blood are particularly preferred targets for the vaccines of the mentioned herein. The vaccines mentioned herein are particularly efficacious against tick species. Examples of such targeted tick species are *Rhipicephalus appendiculatus, R. sanguineus, R. bursa, Amblyonuna variegatum, A. americanum, A. cajennense, A hebraeum, Boophilus microplus, B. annulatus, B. decoloratus, Dermacentor reticulatur, D. andersoni, D. marginatus, D. variabitis, Haemaphysalis inermis, Ha. leachii, Ha. punctata, Hyalomma anatolicum anatolicum, Hy. dromedarii, Hy. marginatum marguiatum, Ixodes ricinus, I. persulcatus, I. scapularis, I. hexagonus, Argas persicus, A. reflexus, Ornithodoros erraticus, O. moubata moubata, O. m. porcinus, and O. savignyi.*

The vaccines mentioned herein are also particularly efficacious against mosquito species. Examples of targeted mosquito species are those of the *Culex, Anopheles* and *Aedes* genera, particularly *Culex quinquefasciatus, Aedes aegypti* and *Anopheles gambiae.*

The vaccines mentioned herein are also particularly efficacious against flea species, such as *Ctenocephalides felis* (the cat flea).

As discussed above, blood-sucking ectoparasites are extremely effective as transmitters of infectious disease. Examples of disease agents, the transmission of which may be prevented or reduced using a vaccine according to the description include those transmitted by mosquitoes, horseflies, sandflies, blackflies, tsetse flies, fleas, lice, mites, flatworms and ticks. Examples of mosquito-borne diseases include Malaria, Dengue Fever, Yellow Fever and Arboviral Encephalitides, including Eastern Equine Encephalitis, Japanese Encephalitis, La Crosse Encephalitis, St. Louis Encephalitis, Western Equine Encephalitis and West Nile Virus Encephalitis and Lymphatic filariasis. Other diseases whose transmission may be prevented include plague (flea); schistosomiasis (flatworms); trypanosomiasis (tsetse fly), Leishmaniasis (sandfly), and Onchocemiasis (blackfly). Examples of tick-transmitted diseases whose transmission may be blocked include protozoan, rickettsial and viral diseases of livestock, including East Coast Fever, babesioses, anaplasmoses, cowdriosis and tick-associated dermatnphilosis, and human diseases, such as Lyme's Disease, Southern Tick-Associated Rash Illness (STARI), Babesiosis, Ehdichiosis, Rocky Mountain Spotted Fever, tularemia, tick-borne relapsing fever, tick-borne encephalitis (TBE) and Crimean-Congo Haemorrhagic Fever. The particular utility of the vaccines described herein in preventing transmission of TBE virus has been illustrated herein.

Preferably, the vaccines described herein are effective against transmission of human diseases. However, these vaccines are also effective against the transmission of diseases in mammals (particularly livestock), birds, reptiles and fish.

By "64p" protein is meant a protein comprising the sequence presented in Figure 1 herein, or a homologue thereof. This protein and its properties are described in detail in co-owned, copending International patent application WO01/80881.

This protein contains at least one immunogenic epitope that is present in all blood-feeding ectoparasites that have been tested so far. It thus appears as if one single vaccine composition may be effective as a broad spectrum vaccine against all of the ectoparasite species that produce a protein containing this common epitope. The availability of a single vaccine that is effective against a number of different ectoparasite species will reduce the cost of administering the vaccine and will thus be advantageous over currently available vaccines.

The sequence in Figure 1 is the 64p sequence from the tick *Rhipicephalus appendiculatus.* This protein possesses a sequence typical of a structural protein, and appears to be secreted in the saliva of ticks. The sequence comprising the first 40 amino acids of the cement protein is strongly collagen-like, whereas the rest of the sequence resembles keratin. Homology searches conducted with the sequence of this protein reveals that the highest level of homology for all searched sequences in the Genbank database (http://www.ncbi.nlm.nih.gov) was 51%, for mouse epidermal keratin subunit L The protein is glycine-rich and contains several repeats of the motif (C/S) 1-4 (Y/F), resembling structural proteins from *Drosophila melanogaster* (cuticular protein) and other insect egg shells, as well as vertebrate cytokeratins including mammalian keratin complex 2 basic protein, mouse keratin, human keratin, collage type IV alpha, and IPIB2 precursor.

The term "homologue" is meant to include reference to paralogues and orthologues of the 64p sequence explicitly identified herein, including, for example, the 64p protein sequence from other tick species, including *R. sanguineus, R. bursa, Amblyomma variegatum, A. americanum, A. cajennense, A. hebraeum, Boophilus microplus, B. annulatus, B. decoloratus, Dermacentor reticulatus, D. andersoni, D. marginatus, D. variabilis, Haemaphysalis inermis, Ha. leachii, Ha. punctata, Hyalomma anatolicum anatolicum, Hy. dromedarii, Hy. marginatum marginatum, Ixodes ricinus, I. persulcatus, I. scapularis, I. hexagonus, Argas persicus, A. reflexus, Ornithodoros erraticus, O. moubata moubata, O. m. porcinus, and O. savignyi.* The term "homologue" is also meant to include the 64p protein sequence from mosquito species, including those of the *Culex, Anopheles* and *Aedes* genera, particularly *Culex quinquefasciatus, Aedes aegypti* and *Anopheles gambiae*; flea species, such as *Ctenocephalides felis* (the cat flea); horseflies; sandflies; blackflies; tsetse flies; fleas; lice; mites; leeches; and flatworms.

Methods for the identification of homologues of the 64p protein will be clear to those of skill in the art. For example, homologues may be identified by homology searching of sequence databases, both public and private. Conveniently, publicly available databases may be used, although private or commercially-available databases will be equally useful, particularly if they contain data not represented in the public databases. Primary databases are the sites of primary nucleotide or amino acid sequence data deposit and may be publicly or commercially available. Examples of publicly-available primary databases include the GenBank database (http://www.ncbi.nlm.nih.gov/), the EMBL database (http://www.ebi.ac.uk/), the DDBJ database (http://www.ddbj.nig.ac.jp/), the SWISS-PROT protein database (http://expasy.hcuge.ch/), PIR (http://pir.georgetown.edu/), TrEMBL (http://www.ebi.ac.uk/), the TIGR databases (see http://www.tigr.org/tdb/index.html), the NRL-3D database (http://www.nbrfa.georgetown.edu), the Protein Data Base (http://www.rcsb.org/pdb), the NRDB database (ftp://ncbi.nlm.nih.gov/pub/nrdb/README), the OWL database (http://www.biochem.ucl.ac.uk/bsm/dbbrowser/OWL/) and the secondary databases PROSITE (http://expasy.hcuge.ch/sprot/prosite.html), PRINTS (http://iupab.leeds.ac.uk/bmb5dp/prints.html), Profiles (http://ulrec3.unil.ch/software/PFSCAN_form.html), Pfam (http://www.sanger.ac.uk/software/pfam), Identify (http://dna.stanford.edu/identify/) and Blocks (http://www.blocks.fhcrc.org) databases. Examples of commercially-available databases or private databases include PathoGenome (Genome Therapeutics Inc.) and PathoSeq (Incyte Pharmaceuticals Inc.).

Typically, greaser than 30% identity between two polypeptides (preferably, over a specified region) is considered to be an indication of functional equivalence and thus an indication that two proteins are homologous. Preferably, proteins that are homologous to the 64p protein have a degree of sequence identity with the 64p protein, or with active fragments thereof, of greater than 55%. More preferred homologues have degrees of identity of greater than 40%, 50%, 60%, 70%, 80%, 90%, 95%, 98% or 99%, respectively with the 64p protein, or with active fragments thereof. Percentage identity, as referred to herein, is as determined using BLAST version 2.1.3 using the default parameters specified by the NCBI (the National Center for Biotechnology Information; http://www.ncbi.nlm.nih.gov/) [Blosum 62 matrix; gap open penalty=11 and gap extension penalty=1].

Homologues of the 64p protein include mutants containing amino acid substitutions, insertions or deletions from the wild type sequence, provided that the immunogenicity of the wild type protein sequence is retained. Mutants thus include proteins containing conservative amino acid substitutions that do not affect the function or activity of the protein in an adverse manner. This term is also intended to include natural biological variants (e.g. allelic variants or geographical variations within the species from which the tissue cement proteins are derived). Mutants with improved immunogenicity from that of the wild type protein sequence may also be designed through the systematic or directed mutation of specific residues in the protein sequence.

For the avoidance of doubt, also embraced by the term "homologues" are those proteins whose encoding genes have not yet been currently cloned, but which are cloned in the future. Methods for cloning genes that are homologues of the proteins mentioned herein will be known to those of skill in the art. For example, a nucleic acid molecule from the gene encoding 64p from *R. appendiculatus* (see Figure 1) as described above may be used as a hybridization probe for RNA, cDNA or genomic DNA isolated from an ectoparasite, in order to isolate full-length cDNAs and genomic clones encoding the equivalent 64p protein in this species. In this regard, the following techniques, among others known in the art, may be utilised and are discussed below for purposes of illustration.

One such method is to probe a genomic or cDNA library with a natural or artificially-designed probe using standard procedures that are recognised in the art (see, for example, "Current Protocols in Molecular Biology", Ausubel et al. (eds). Greene Publishing Association and John Wiley Interscience, New York, 1989,1992). Probes comprising at least 15, preferably at least 30, and more preferably at least 50, contiguous bases that correspond to, or are complementary to, nucleic acid sequences from an appropriate encoding gene, such as that set out in Figure 1 herein.

Such probes may be labelled with an analytically-detectable reagent to facilitate their identification. Useful reagents include, but are not limited to, radioisotopes, fluorescent dyes and enzymes that are capable of catalysing the formation of a detectable product. Using these probes, the ordinarily skilled artisan will be capable of isolating complementary copies of genomic DNA, cDNA or RNA polynucleotides encoding proteins of interest from human, mammalian or other animal sources and screening such sources for related sequences, for example, for additional members of the family, type and/or subtype.

In many cases, isolated cDNA sequences will be incomplete, in that the region encoding the polypeptide will be cut short, normally at the 5' end. Several methods are available to obtain full length cDNAs, or to extend short cDNAs. Such sequences may be extended utilising a partial nucleotide sequence and employing various methods known in the art to detect upstream sequences such as promoters and regulatory elements. For example, one method which may be employed is based on the method of Rapid Amplification of cDNA Ends (RACE; see, for example, Frohman et al., Proc. Natl. Acad. Sci. USA (1988) 85: 8998-9002). Recent modifications of this technique, exemplified by the Marathon™ technology (Clontech Laboratories Inc.), for example, have significantly simplified the search for longer cDNAs. A slightly different technique, termed "restriction-site" PCR, uses universal primers to retrieve unknown nucleic acid sequence adjacent a known locus (Sarkar, G. (1993) PCR Methods Applic. 2:318-322). Inverse PCR may also be used to amplify or to extend sequences using divergent primers based on a known region (Triglia, T., et al. (1988) Nucleic Acids Res. 16:8186). Another method which may be used is capture PCR which involves PCR amplification of DNA fragments adjacent a known sequence in human and yeast artificial chromosome DNA (Lagerstrom, M. et al. (1991) PCR Methods Applic. 1: 111-119). Another method which may be used to retrieve unknown sequences is that of Parker, J.D. et al. (1991); Nucleic Acids Res. 19:3055-3060). Additionally, one may use PCR, nested primers, and PromoterFinder^{™} libraries to walk genomic DNA (Clontech, Palo Alto, CA). This process avoids the need to screen libraries and is useful in finding intron/exon junctions.

When screening for full-length cDNAs, it is preferable to use libraries that have been size-selected to include larger cDNAs. Also, random-primed libraries are preferable, in that they will contain more sequences that contain the 5' regions of genes. Use of a randomly primed library may be especially preferable for situations in which an oligo d(T) library does not yield a full-length cDNA. Genomic libraries may be useful for extension of sequence into 5' non-transcribed regulatory regions.

Methods for DNA sequencing and analysis are of course well known and are generally available in the art. Preferably, the sequencing process may be automated using machines such as the Hamilton Micro Lab 2200 (Hamilton, Reno, NV), the Peltier Thermal Cycler (PTC200; MJ Research, Watertown, MA) and the ABI Catalyst and 373 and 377 DNA Sequencers (Perkin Elmer).

Fragments of the 64p protein are also useful as components of the vaccines described herein. Included as such fragments are not only fragments of the *Rhipicephalus appendiculatus* 64p protein that is explicitly identified herein in Figure 1, but also fragments of homologues of this protein. Such homologous fragments will typically possess greater than 30% identity with the *R. appendiculatus* 64p sequence, although more preferred homologues will display degrees of identity of greater than 40%, 50%, 60%, 70%, 80%, 90%, 95%, 98% or 99%, respectively with the 64p protein fragments that are explicitly identified herein.

For example, short stretches of peptide derived from immunogenic portions of 64p proteins may be particularly useful as immunogens. Such short stretches of polypeptide sequence are simple to produce in large quantities, either synthetically or through recombinant means. Protein fragments may in many instances be preferred for use in the vaccines described herein, since these fragments are likely to fold into conformations not adopted by the full length wild type 64p sequence. Since some 64p proteins are likely to have evolved so as to resemble the tissues of the host skin and thus to avoid provoking a host immune response against the tick, such unnatural forms of tick cement proteins that expose 'hidden' epitopes are likely to be of particular use in the vaccines described herein.

Examples of fragments of 64p proteins that are useful for inclusion in the vaccine compositions mentioned herein include various fragments that have been generated recombinantly by the inventors (see WO01/80881), and homologues of these fragments and mutants of the kind discussed above. As will be apparent to the skilled reader, similar fragments to those that are explicitly disclosed herein may be prepared from ectoparasite species other than ticks.

The details of the *R. appendiculatus* fragments described herein are as follows.

The fragment termed 64trp1 is a small soluble C-terminal fragment of the 64P protein consisting of 29 amino acids (residues 103-132 inclusive of the sequence of Figure 1) cloned as a glutathione-S-transferase (GST) / histidine tag fusion protein with a molecular weight of around 30 kDa.

The fragment termed 64trp2 refers to a small soluble N-terminal fragment of the 64P protein consisting of 51 amino acids (residues 1-51 inclusive of the sequence of Figure 1) cloned as a glutathione-5-transferase (GST) / histidine tag fusion protein with a molecular weight of around 33 kDa.

The fragment termed 64trp3 refers to a larger soluble N-terminal fragment of 64P protein consisting of 70 amino acids (residues 1-70 inclusive of the sequence of Figure 1) cloned as a glutathione-5-transferase (GST) / histidine tag fusion protein with a molecular weight of around 36 kDa.

The fragment termed 64trp4 is a soluble C-terminal fragment of 64P protein consisting of 63 amino acids (residues 69-132 inclusive of the sequence of Figure 1) cloned as a glutathione-S-transferase (GST) / histidine tag fusion protein with a molecular weight of around 35 kDa.

The fragment termed 64trp5 is the full-length clone of 64P protein sequence consisting of 133 amino acids cloned as a GST fusion protein (i.e. minus the histidine tag). This protein is soluble and has a molecular weight of 41 kDa.

The fragment termed 64trp6 refers to the full-length clone of 64P protein consisting of 133 amino acids cloned as a glutathione-S-transferase (GST) / histidine tag fusion protein. This fragment is insoluble and has an approximate molecular weight of around 42 kDa.

These protein fragments, and homologues thereof, are particularly preferred components for corporation in the vaccines mentioned herein. These fragments may be expressed as soluble protein, or may alternatively be expressed in inclusion bodies and purified under denaturing conditions. For example, the construct 64op6 as isolated from *R. appendiculatus* has been prepared as a denatured protein expressed in inclusion bodies and demonstrated to be immunogenic in this form.

Immunisation with these protein fragments, followed by attachment of ectoparasite, results in inflammation at the attachment site and subsequent death of the ectoparasite. The skilled reader will appreciate that the presence of the heterologous GST and HIS tag sequences is purely for convenience of protein production. These stretches of sequence are not considered to be essential to this aspect.

Conveniently, the vaccines according to the description contain a 64p protein, fragment thereof or homologue thereof, expressed in recombinant form. Recombinantly-expressed protein is inexpensive to produce and, using the now standard techniques of genetic engineering, allows the simple manipulation of gene sequences to give a desired protein product.

It is preferred that the vaccines of the description are effective against both adult and immature forms of the ectoparasite. The term "immature" is meant to include both nymph and larval forms of the ectoparasite. This means that the whole ectoparasite population may be targeted using the vaccine, so increasing the efficiency of eradication of ectoparasite and infectious disease causing agent. The vaccines may specifically target adult or immature forms of ectoparasites, but will preferably target all parasitic stages of the life cycle. Of the fragments specifically exemplified herein, 64trp2-, 64trp3-, 64trp5- and 64trp6-immunised animals has been found to cause significant mortality in tick nymphs or adult ticks or both nymphs and adults, depending on the tick species, and these fragments are thus particularly preferred.

According to a further embodiment, there is provided a cocktail vaccine comprising, in addition to the 64p protein, fragment or homologue, a second active agent.

The second active agent may preferably be a second immunogenic protein, or protein fragment derived from a blood-feeding ectoparasite. More preferably, the second immunogenic protein, fragment or homologue is a 64p protein or protein fragment.

The second active agent may be a vaccine against an infectious disease. It has been found that whilst certain commercially available vaccines against infectious disease are effective in abrogating the effects on the host of the virus that causes the infectious disease and thus in protecting against lethal challenge with virus-infected ticks, these vaccines do not protect the host against infection. This is shown herein (see Example 3.10) by the ability of immunised hosts to support virus transmission to uninfected ticks feeding upon them.

The cocktail vaccine produced by the combination of a 64p-based vaccine according to the description and a vaccine against an infectious disease would be effective in preventing viral transmission through the properties of the 64p components described above, and would also protect against lethal viral challenge through the properties of the vaccine against infectious disease. Such a combined vaccine should thus protect against both host infection and death.

Preferably, the vaccine against an infectious disease used as the second agent is a vaccine against TBE. Examples of commercially-available TBE vaccines are known to those of skill in the art.

Optionally, the cocktail vaccine may contain an adjuvant.

For example, any two or more immunogenic 64p proteins, protein fragments or functional equivalents may be used as components of such a cocktail vaccine, and may be from different or from the same tick species. For example, it may be desired to generate a vaccine that specifically targets more than one ectoparasite, or that targets different proteins from the same ectoparasite. In this manner, it may be possible to generate a more efficacious vaccine with greater species coverage. Particularly preferred combinations of components include the combination of 64trp2, 64trp3, 64trp5 and 64trp6, the combination of 64trp2 and 64trp5, the combination of 64trp2 and 64trp6 and the combination of 64trp5 and 64trp6. These combinations are demonstrated herein to possess particular efficacy in targeting both adult and immature ticks, in conferring cross-species resistance and in blocking the transmission to the host of the disease-causing agent.

Vaccine compositions according to the description may also comprise additional agents, for example, molecules that the ectoparasite uses to promote pathogen transmission, such as interferon regulators, complement inhibitors, chemokine regulators and immunoglobulin-binding proteins. In this way, other bioactive molecules that are released from the salivary glands of ectoparasites may be recognised as foreign by the host immune system and an immune response mounted.

A further aspect of the present description comprises a vaccine containing a 64p protein, fragment or homologue fused to another molecule, such as a label, a toxin or other bioactive or immunogenic molecule. Particularly suitable candidates for fusion may be a molecule such as glutathione-S-transferase or a histidine tag, although luciferase, green fluorescent protein or horse radish peroxidase may also be suitable. Linker molecules such as streptavidin or biotin may also be used, for example, to facilitate purification of the cement protein.

Fusion proteins may be created chemically, using methods such as chemical cross-linking. Such methods will be well known to those of skill in the art and may comprise, for example, cross-linking of the thiol groups of cysteine residues. Chemical cross-linking will in most instances be used to fuse tissue cement proteins to non-protein molecules, such as labels.

When it is desired to fuse a tissue cement protein to another protein molecule, the method of choice will generally be to fuse the molecules genetically. In order to generate a recombinant fusion protein, the genes or gene portions that encode the proteins or protein fragments of interest are engineered so as to form one contiguous gene arranged so that the codons of the two gene sequences are transcribed in frame.

Immunisation with naked, plasmid DNA encoding specific antigens has recently been acknowledged as an efficient method of presenting antigens to the mammalian immune system, resulting in strong humoral and cellular immune response (Ulmer et al., Science 1993, 259, 1745-1749). This technique, also referred to as DNA vaccination, has been successfully applied to generate antibodies directed against several proteins derived from viruses (Ulmer *et al., loc cit*.; Cox et al., J. Virol. 1993, 67, 5664-5667; Fynan et al., Proc. Natl. Acad. Sci. USA 1993, 90, 11478-11482; Robinson et al., Vaccine 1993, 11, 957-960; Wang et al., 1993, DNA Cell Biol. 1993, 12, 799-805; Davis et al., Hum. Mol. Genet. 1993, 2, 1847-1851; Xiang et al., Virology 1994, 199, 132-140; Xiang et al., Virology 1995, 209, 569-579; and Justewicz et al., J. Virol. 1995, 69, 7712-7717), parasites (Sedegah et al., Proc. Natl. Acad. Sci. USA 1994, 91, 9866-9870; Mor et al., J. Immunol. 1995, 155, 2039-2046; and Yang et al., Biochem. Bioph. Res. Comm. 1995, 212, 1029-1039) and bacteria (Anderson et al., Infect. Immun. 1996, 64, 3168-3173), and, in several cases, a significant protective response has been elicited by the host. These DNA vaccines continuously stimulate the immune system, amplifying immunity and thereby reducing the cost of production and delivery as no booster injections are required.

Based on the available evidence, immunisation with plasmid DNA encoding the various 64p proteins is likely to be a useful technique to improve their anti-tick vaccine effects further. The method would involve direct injection of the host with a eukaryotic expression vector such that one or more 64p proteins are expressed by *in vivo* transcription then translation of the corresponding sequence within the vaccinated host (humans, livestock, or other animals). The vaccines of any one of the above-described aspects may additionally comprise an adjuvant. Suitable adjuvants to enhance the effectiveness of the immunogenic proteins according to the present description include, but are not limited to, oil-in-water emulsion formulations (optionally including other specific immunostimulating agents such as muramyl peptides or bacterial cell wall components), such as for example (a) those formulations described in PCT Publ. No. WO 90/14837. Other suitable adjuvants will be known to those of skill in the art and include Saponin adjuvants, such as TiterMax Gold (CytRx Corporation, 150 Technology Parkway, Atlanta Norcross, Georgia), Stimulon^{™} (Cambridge Bioscience, Worcester, MA), ISA Montanide 50, cytokines, such as interleukins, interferons, macrophage colony stimulating factor (M-CSF) or tumor necrosis factor (TNF).

According to a further embodiment of the description, there is provided a monoclonal antibody that is reactive with a 64p protein and which is thus effective in blocking the transmission of a disease-causing agent. By "reactive" is meant that the antibody binds to one or more epitopes of a 64p protein with an affinity of at least 10⁻⁸M, preferably at least 10⁻⁹M, more preferably at least 10⁻¹⁰M. According to a preferred embodiment of this aspect, - the antibody or antiserum is reactive against analogues of the 64p protein, for example, 64p protein analogues from a number of different ectoparasite species, such as the tick, mosquito, sandfly, blackfly and so on. This aspect of the description includes a method for the production of such an antibody or an antiserum, comprising immunising an animal with a 64p protein, fragment thereof, or homologue thereof as listed in any one of the above-described aspects.

According to a still further aspect of the description, there is provided a process for the formulation of a vaccine composition comprising bringing a 64p protein, fragment or homologue into association with a pharmaceutically-acceptable carrier, optionally in conjunction with an adjuvant. The technology referred to as jet injection (see, for example, www.powderject.com) may also be useful in the formulation of vaccine compositions. According to a still further aspect of the present description there is provided a method of immunising a mammal against an ectoparasite-transmitted disease or against a blood-feeding ectoparasite, comprising administering to an animal, a vaccine according to any one of the above-described aspects, - Such an immunisation method may utilise conventional means, but alternative methods of administering vaccines, such as through the use of jet injection may be equally effective or even preferable (see, for example, www.powderject.com; also Sarno et al. (2000) Pediatr. Infect. Dis. J. 19:839-842).

The invention also provides a 64p protein, fragment thereof or homologue thereof, for use in a vaccine. The invention further provides for the use of a 64p protein, fragment thereof or homologue thereof as a component of a vaccine.

Various aspects and embodiments of the present invention will now be described in more detail by way of example, with particular reference to the 64p protein from the tick, *Rhipicephalus appendicaulatus*. It will be appreciated that modification of detail may be made without departing from the scope of the invention.

### Brief description of the Figures

Figure 1: Nucleotide and inferred amino acid sequences of 64p: Complete cDNA sequence and cDNA-inferred protein sequence of clone 64. The putative signal sequence is given in bold. A possible glycosaminoglycan attachment site is underlined. The first 40 amino-acid piece of the mature protein is collagen-like, the remainder of the sequence resembles keratin. The protein is glycine-rich and contains several repeats of the motif (C/S)1-4(Y/F), which is also found in structural proteins from insect egg shells. The tyrosines may be involved in cross-linking by formation of dityrosine-bridges by phenoloxidases. * indicates the stop codon.

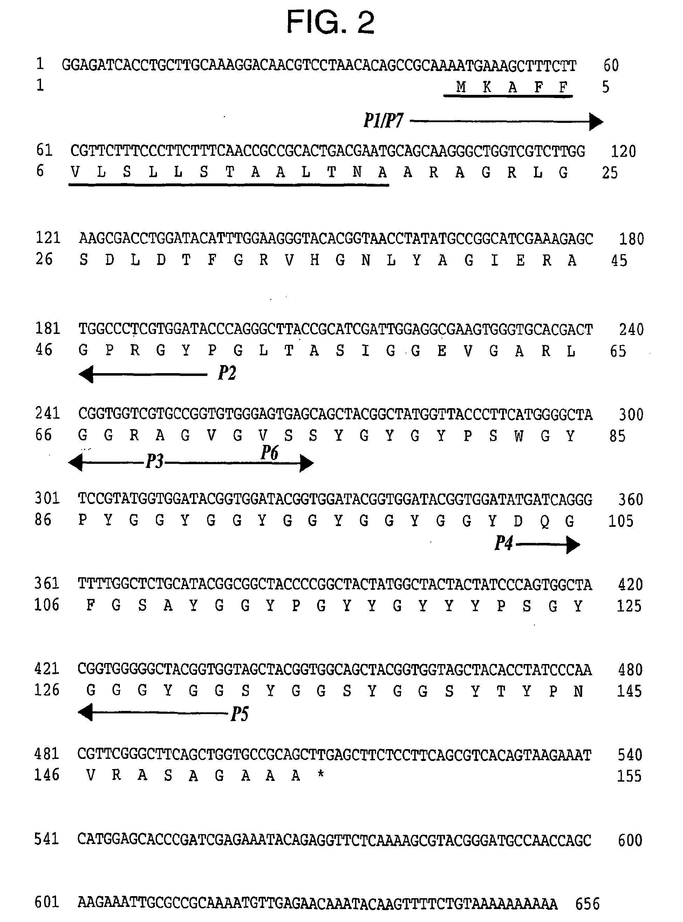

Figure 2: Amino acid sequences of 64P protein fragments (64TRPs) expressed in *Escherichia coli*. P1/ P2, P1/P3, P4/P5, P6/P5, P1/P5 and P7/P5 refer to primers used to subclone PCR products from 64P amino acid sequence into the plasmid pGEX-2T, for expression in *Escherichia coli* cells as truncated versions of 64P protein, i.e. 64trp2 (51 amino acids), 64trp3 (70 amino acids), 64trp1 (29 amino acids), 64trp4 (63 amino acids), 64trp5 (133 amino acids without HIS.TAG) and 64trp6 (133 amino acids with HIS.TAG), respectively. Predicted possible cleavage signal peptide (amino acids 1 to 18) is underlined in green.

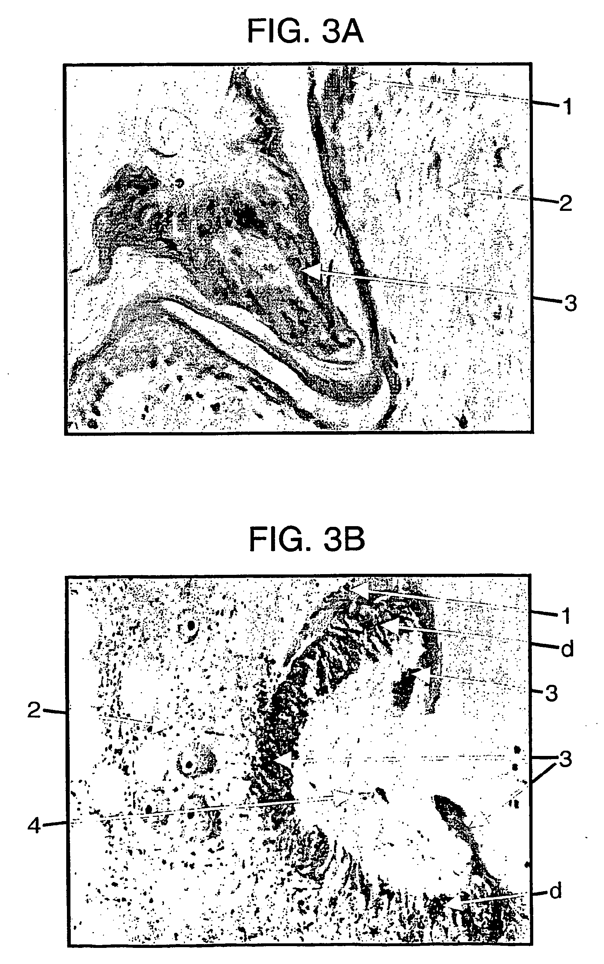

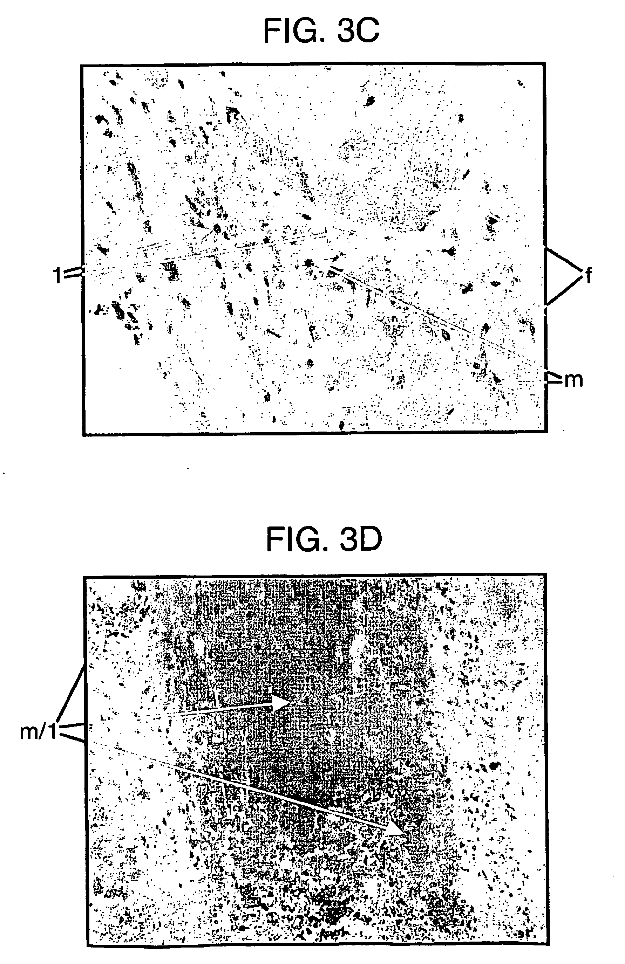

Figure 3: Histological studies of skin sections from hamsters immunised with different cocktails of 64trp proteins, post-feeding of *Ixodes ricinus* nymphs, stained with Haematoxylin and Eosin. (A)= histological section from skin of GST-immunised, control hamster & (B), (C) & (D)= histological sections from skin of hamsters immunised with 64trp proteins, post-feeding with *I. Ricinus* nymphs, stained with haematoxylin & eosin stains; sections (A), (B) & (D) - magnification=20X; section (C) - magnification=40X; arrows: 1=epidermis, 2=dermis, 3 =subcutis & 4=cement cone; d=dentritic-like cells (i.e. Langerhan cells on cement cone), f=fibroblasts, l=lymphocytes & m=mast cells.

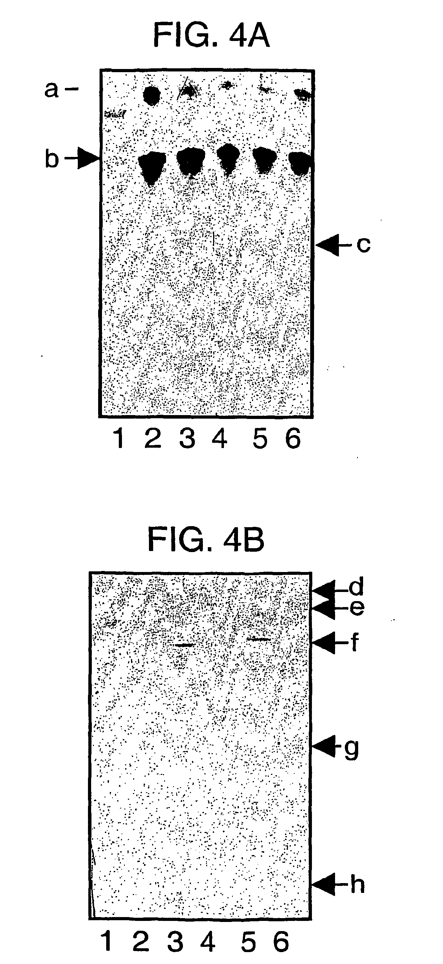

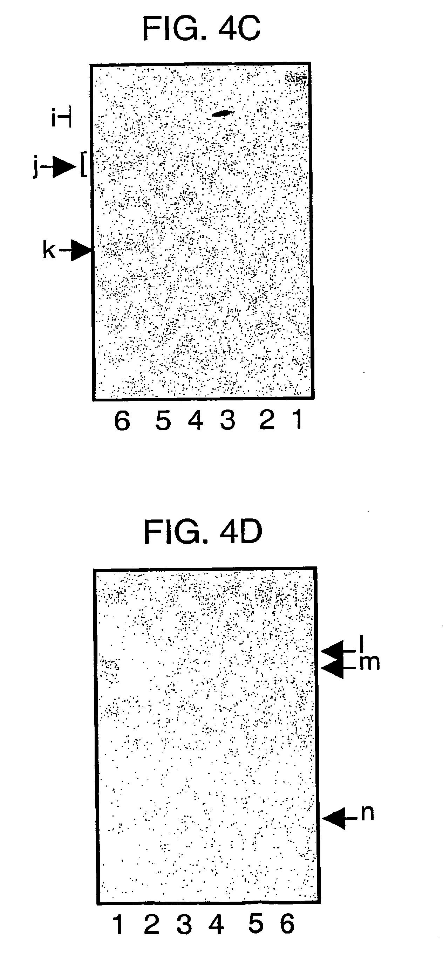

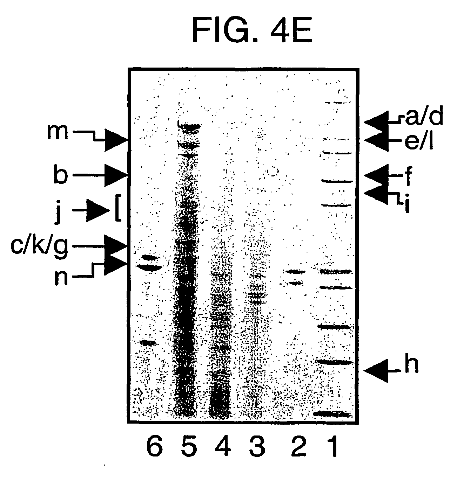

Figure 4: Immunoblots of different mosquito antigens probed with **(A)** anti-64trp2, **(B)** anti-64trp5, **(C)** anti-64trp6 and **(D)** anti-GST(control) sera, respectively; **(E)** Coomassie Blue stained 4-12% gradient gel showing the protein profile of the same mosquito antigens.

Lanes 2, 3, 4, 5 & 6 in all figures denote: **Lane 2,** salivary gland extract of *Anopheles gambiae*, **Lane 3** midgut extract of *Anopheles gambiae*, **Lane 4** midgut extract of *Aedes aregypti*, **Lane 5** midgut extract of *Culex quinquefasciatus* and **Lane 6** salivary gland extract of *Culex quinquefasciatus*, mosquitoes. Immunopositive bands labelled as **a - n;** faintly visible bands **l - n** refer to non-specific binding of proteins in the mosquito tissue extracts by the anti-GST control serum.

Lane **1** figures **A, B, C & D:** SeeBlue^{™} Plus2 protein molecular weight markers: 188 kD = Myosin, 98 kD = Phosphorylase B, 62 kD = Bovine serum albumin, 49 kD = Glutamic dehydrogenase, 38 kD = Alcohol dehydrogenase, 28 kD = Carbonic anhydrase, 17 kD = Myoglobin Red and 14 kD = Lysozyme; lane **1** figure **E:** Mark 12 protein markers: 200 kD = Myosin, 116.3 kD = *B*-galactosidase, 97.4 kD = Phosphorylase b, 66.3 kD = Bovine serum albumin, 55.4 kD = Glutamic dehydrogenase, 36.5 kD = Lactate dehydrogenase, 31 kD = Carbonic anhydrase, 21.5 kD = Trypsin inhibitor, 14.4 kD = Lysozyme and 6 kD = Aprotinin.

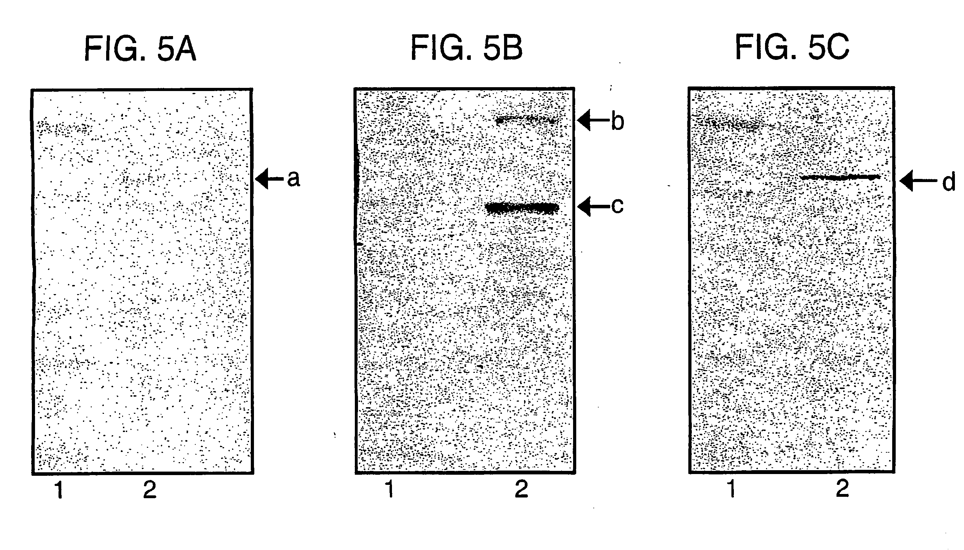

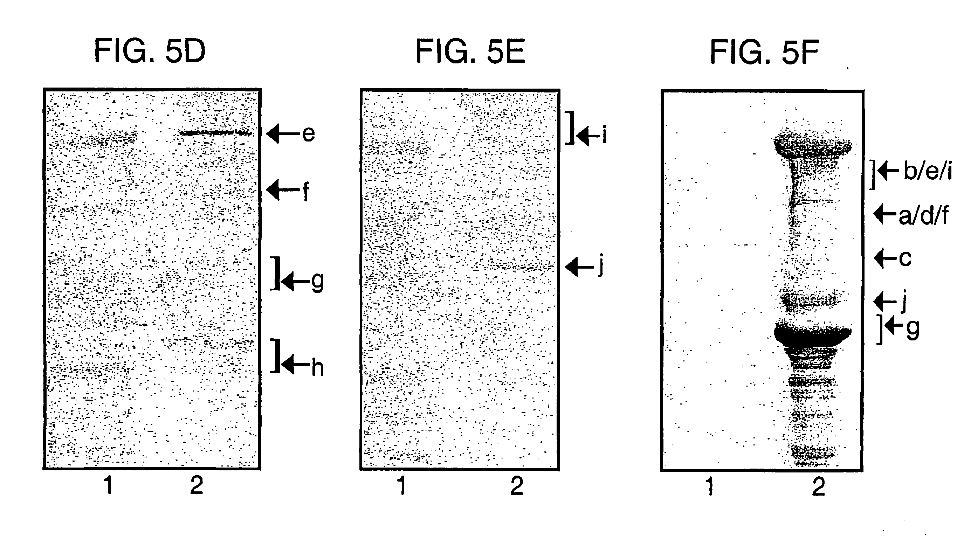

Figure 5: Cross-reactivity between *Ctenocephalides felis* (cat flea) antigens using antisera from guinea pigs immunised with recombinant R. appendiculatus 64trp proteins. **A, B, C, D** & **E** immunoblots of whole flea extracts of *Ctenocephalides felis* fed on cats using GST **(A),** 64trp2 **(B),** 64trp3 **(C),** 64trp5 **(D)** and 64trp6 **(E)** antisera, respectively; **F** Coomassie Blue stained 4-12% Bis-Tris gradient gel (NuPAGE-Novex) of the same extracts.

Lanes: **A/1, B/1, C/1, D/1** & **E/1:** See Blue^{™} Plus 2 protein molecular weight markers (Novex): 98 kD=Phosphorylase b, 62 kD=BSA, 49 kD=Glutamine dehydrogenase, 38 kD=Alcohol dehydrogenase, 28 kD=Carbonic anhydrase, 17 kD=Myoglobin Red & 14 kD=Lysozyme. Lane: **F/1:** Mark 12^{™} protein moleculat weight markers 9Novex): 200 kD=Myosin, 116.3 kD=B-galactosidase, 97.4 kD=Phosphorylase b, 66.3 kD=BSA, 55.4 kD=Glutamic dehydrogenase, 36.5 kD=Lactate dehydrogenase, 31 kD=Carbonic anhydrase & 21.5 kD=Trypsin inhibitor.

Lanes: **B/2, C/2, D/2** & E/2= *C. felis* whole extract of which immuno-positive bands were observed as b=110 kD, c=62 kD, d=75 kD, e=98 kD, f=75 kD, g=48-50 kD, h=28-31 kD, i=98-120 kD and j=50 kD, respectively. Lane: A/2= C. *felis* whole extract showing a faint immuno-positive band observed as a, due cross-reactivity of flea antigens with anti-GST antiserum.

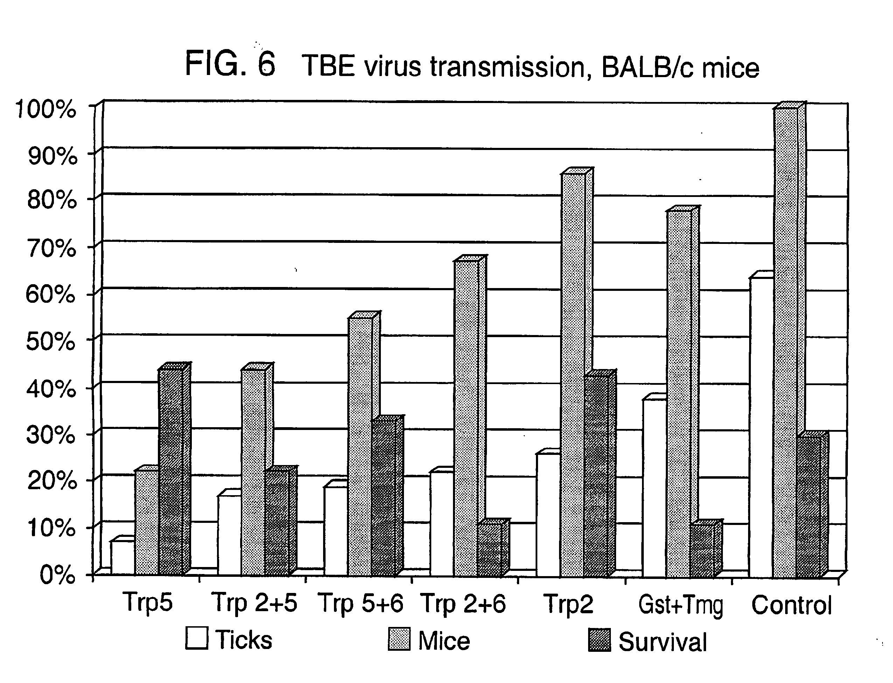

Figures 6-8: Effect of 64TRP constructs in blocking TBE virus transmission in mice.

### Examples

Expression of truncated cement proteins (64TRP) in bacteria is reported in WO01/80881,

This patent application also details the expression of the various 64TRP constructs, along with immunohistochemical studies using antiserum to 64TRP, and vaccination trials in Dunkin-Hartley Guinea pigs. It was also reported in this application that antisera raised against *Rhipicephalus appendiculatus* cement protein 64TRP were cross-reactive with antigenic epitopes in the salivary gland, midgut and haemolymph of adult female *R. appendiculatus* and with antigenic epitopes in the salivary glands, midgut and haemolymph of three other ixodid tick species. The results suggested that the candidate vaccine(s) derived from *R. appendiculatus* cement provide a broad spectrum vaccine that is effective against a number of different tick species.

On the basis of the observed cross-reactivities, 64trp6 of *R. appendiculatus* was selected as an immunogen for a vaccine trial. The results presented in this specification showed that putative vaccines derived against cement protein 64TRP of *R. appendiculatus* were cross-protective against adults and nymphs of *R. sanguineus*.

Following on from this work, additional work has now been performed that has revealed surprising findings relating to the cross-reactivity of 64TRP-based vaccines in insects, and the ability of these vaccines to confer resistance to infection.

### Example 1: Cross-reactivity and Cross-protection Vaccine Trial with insects

### 1.1 Selection of immunogens

Candidate immunogens were identified on the basis of whether antiserum to the construct detected specific cross-reacting antigens in extracts of mosquitoes and fleas.

Cross-reactivity studies using immunoblotting with 64trp antisera showed detection of protein bands with mosquito extracts of *Anopheles gambiae* salivary gland and midgut, *Aedes aegypti* midgut, and *Culex quinquefasciatus* salivary gland and midgut (Figure 3).

Using 64trp2 antiserum (Figure 4A), two major bands (**a** and **b**) were detected in all extracts.

Antisera to 64 trp5 (Figure 4B) detected several less pronounced bands in midgut of *An. gambiae* and *C. quinquefasciatus*. Antiserum to 64trp6 detected a prominent band in *An. gambiae* midgut (Figure 4C).

The control antiserum raised against GST detected very faint bands in midgut of An. *gambiae* and *C. quinquefasciatus* that differed in size from those detected with 64trp antisera (Figure 4D).

Based on the availability of *A. aegypti* larvae, and the observed cross-reactivities, a vaccine trial was undertaken using 64trp2 and 5.

### 1.2 Treatments for vaccine trial:

- Group 1: Recombinant 64trp2 + 64 trp5 + Montanide ISA (2 mice)
- Group 2: Recombinant 64trp5 + Montanide ISA (2 mice)
- Group 3: Recombinant 64trp2 + Montanide ISA (2 mice)
- Group 4: GST (control) (2 mice)
- Group 5: Recombinant 64trp2 + 64 trp5 + Montanide ISA (2 guinea pigs)
- Group 6: Recombinant 64trp2 + Montanide ISA (2 guinea pigs)
- Group 7: Recombinant 64trp5 + Montanide ISA (2 guinea pigs)
- Group 8: GST (control) (2 guinea pigs)
Total Number of Animals = 8 mice + 8 guinea pigs

### 1.3 Route and dose:

Subcutaneous inoculation in the prescapular region either singly or as combined antigens into a single site.

Dose 50 µg antigen per guinea pig and 10 µg antigen per mouse.

### 1.4 Vaccination scheme:

1. Primer inoculation
2. First boost
3. Test bleed at 10 to 12 days post-inoculation
4. Second boost (if antibody titre <1/5000)
5. Test bleed at 10 to 12 days post-inoculation
6. Antibody titre >1/5000: challenge with *Aedes aegypti* mosquitoes.
7. Evaluate local inflammatory immune response to repeated mosquito feeding, and survival of fed mosquitoes.

### 1.5 Results

The results are summarised in Tables 1a and 1b. Overall, they show that putative vaccines derived against cement of *R. appendiculatus* were cross-reactive against *A. aegypti* mosquitoes.

### (i) Feeding success

Adult female mosquitoes fed preferentially on young (5-6 week-old) mice compared with guinea pigs.

### (ii) Inflammatory response

Local skin hypersensitivity reactions, observed as intense papular swellings, were observed on the abdomen and foot pads of mice by the third feeding.

### (iii) Post-feeding mortality

Higher mortality was observed among mosquitoes exposed to 64trp-immunised animals compared to control animals.

### (iv) Antibody titres

Antibody titres for Balb/c mice immunised with 64trp2 were > 1:64,000 and for C57/BI10 mice immunised with 64trp2, 1:64,000.

### 1.6 Conclusions

1. Antibodies raised against the 64trp proteins cross-react in immunoblots with antigenic epitopes in salivary gland and midguts of adult mosquitoes.
2. A host inflammatory response was observed in mice immunised with 64trp immunogens.
3. The mortality in mosquitoes fed on 64trp-immunised animals indicates that the tick cement protein is a candidate for developing anti-mosquito vaccines.

**Table 1a. Effect of feeding Aedes aegypti mosquitoes on mice immunised with recombinant 64trp proteins**

| Mouse strain | 64trp protein | Live mosquitoes | | Dead mosquitoes | | Total MM+FF | Mortality (%) FF |
|---|---|---|---|---|---|---|---|
| | | MM | FF | MM | FF | | |
| Balb/C | 64trp2/5 | 19 | 9 | 0 | 5* | 33 | 20.8 |
| Balb/C | 64trp5 | 14 | 13 | 0 | 8 | 35 | 30.1 |
| Balb/C | 64trp2 | 6 | 10 | 0 | 7* | 23 | 41.2 |
| Balb/C | GST (control) | 19 | 16 | 0 | 0 | 35 | 0 |
| C57/BI10 | 64trp2/5 | 10 | 15 | 1 | 6* | 33 | 28.6 |
| C57/BI10 | 64trp5 | 8 | 14 | 0 | 2 | 31 | 12.5 |
| C57/BI10 | 64trp2 | 23 | 9 | 0 | 3 | 35 | 25 |
| C57BI10 | GST (control) | 22 | 7 | 0 | 1 | 30 | 12.5 |

**Table 1b. Effect of feeding Aedes aegypti mosquitoes on guinea pigs immunised with recombinant 64trp proteins**

| Guinea pig no. | 64trp protein | Live mosquitoes | | Dead mosquitoes | | Total MM+FF | Mortality (%) FF |
|---|---|---|---|---|---|---|---|
| | | MM | FF | MM | FF | | |
| 1a | 64trp2/5 | 19 | 21 | 0 | 8 | 48 | 27.6 |
| 1b | 64trp2/5 | 26 | 19 | 1 | 6 | 52 | 24 |
| 2a | 64trp2 | 19 | 14 | 0 | 2 | 35 | 12.5 |
| 2b | 64trp2 | 14 | 18 | 0 | 9 | 41 | 33.3 |
| 3a | 64trp5 | 16 | 21 | 1 | 13 | 51 | 38.2 |
| 3b | 64trp5 | 16 | 11 | 0 | 6 | 33 | 35.3 |
| 4a | GST (control) | 18 | 10 | 1 | 0 | 29 | 0 |
| 4b | GST (control) | 19 | 9 | 0 | 1 | 26 | 10 |

| | | | | | | | |
|---|---|---|---|---|---|---|---|
| *female mosquitoes observed dying during counting | | | | | | | |

### Example 2: Antigenic cross-reactivity between Rhipicephalus appendiculatus and Ctenocephalides felis cat flea detected by immunoblotting using antisera to R. appendiculatus cement protein 64 trp constructs

Antisera to 64trp 2, 64trp5, and 64trp6 showed strong cross-reactivities in immunoblots of whole cat flea extract probed with the respective antisera (Figure 5). The results are summarised in Table 2 below. Single cross-reactions were also detected with anti-64trp3 and anti-GST sera. The cross-reactivities demonstrate the potential for developing an anti-flea vaccine using the tick cement protein.

**Table 2. Cross-reactivity between Rhipicephalus appendiculatus and Ctenocephalides felis whole flea extract using sera from guinea pigs immunised with 64 trp recombinant antigens**

| Antiserum | Tick Antigens | | | | | | |
|---|---|---|---|---|---|---|---|
| | ***R. appendiculatus*** | | | | | | ***C. felis*** |
| | **CC** | **SG** | **gut** | **H** | **N** | **L** | |
| **Anti-64trp2 ab'** | | | | | | | |
| **(50a.a. N-term. Frag. of 64P) Effective against RA Adult/nymph ticks (soluble antigen)** | **+** | **+** | **+** | **+** | **+** | **+** | **+** |
| **Anti-64trp3 ab'** | | | | | | | |
| **(70a.a. N-term. Frag. of 64P) Effective against RA Adult/nymphs ticks (soluble antigen)** | **+** | **+** | **+** | **+** | **+-** | **+** | **+** |
| **Anti-64trp5 ab'** | | | | | | | |
| **(133a.a. full- length clone of 64P) effective against RA nymph ticks (soluble antigen)** | **+** | **+** | **+** | **+** | **-** | **-** | **+** |
| **Anti-64trp6 ab'** | | | | | | | |
| **(133a.a. full- length clone of 64P) effective against RA nymph ticks (denatured antigen)** | **-** | **+** | **+** | **+** | **+** | **+** | **+** |
| **nti-GST ab' control antiserum** | **-** | **+** | **-** | **-** | **-** | **-** | **+** |

| | | | | | | | |
|---|---|---|---|---|---|---|---|
| CC = tick cement cone extract; SG = salivary gland extract, gut = midgut extract; H = haemolymph; N = whole nymphal extract; L = whole larval extract; + = positive and - = negative reactions, respectively, to antisera used in immunoblots; ab' = antiserum | | | | | | | |

### Example 3: Evaluation of 64p anti-tick vaccine constructs for their ability to protect mice against tick-borne encephalitis (TBE) virus infection.

### 3. 1 Selection of immunogens

Candidate immunogens were selected on the basis of whether antiserum to the 64TRP constructs detected specific cross-reacting antigens in extracts of *Ixodes ricinus*, the tick vector of TBE virus (see Table 3 of WO01/80881).

### 3.2 Treatments for vaccine trial (Trial 1):

- Group A: Recombinant 64trp2 + TiterMax Gold (TMG) (10 mice)
- Group B: Recombinant 64trp5 + TMG (10 mice)
- Group C: Recombinant 64trp2 + 64trp6 + TMG (10 mice)
- Group D: Recombinant 64trp5 + 64trp6 + TMG (10 mice)
- Group E: Recombinant 64trp2 + 64trp5 + TMG (10 mice)
- Group F: GST protein (10 mice)
- Group G: TMG (10 mice)
- Group H: untreated (10 mice)

Total number of animals = 80 Balb/c mice.

### 3.3 Route and dose:

Subcutaneous inoculation in the prescapular region either singly or as combined antigens into a single site. Final volume of inoculum = 200µl
Group A. 15µl TRP2 + 85µl PBS + 100µl TMG
Group B. 20µl TRP5 + 80µl PBS + 100µl TMG
Group C. 20µl TRP2 + 25µl TRP5 + 55µl PBS + 100µl TMG
Group D. 20µl TRP2 + 5µlTRP6 + 75µl PBS + 100µl TMG
Group E. 25µl TRP5 + 5µl TRP6 + 70µl PBS + 100µl TMG
Group F. 10µl GST + 90µl PBS + 100µl TMG
Group G. 100µl TM + 100µl PBS Group H. untreated control group

### 3.4 Vaccination scheme:

All animals were immunized on the same day.

Serum samples were collected from the half of the exp. mice from sinus orbitalis in each of the 8 groups 12 to 14 days after immunisation (30µl to 50µl serum/animal) and then 25 days after immunisation from all remaining mice.

### 3.5 TBE virus infection and transmission

TBE virus donors used to infect the mice were field collected *Ixodes ricinus* female ticks (FIR) inoculated parenterally with TBE virus (Hypr strain), diluted 10⁻¹, volume 0.002 ml, in total 5000 PFU/tick. They were inoculated on either the same day or the day after immunisation of the mice.

Recipient ticks used to assay for virus transmission were uninfected *Ixodes ricinus* males (MIR) and nymphs (NIR) although male ticks were not tested. Mice were challenged with 1 infected FIR, 1 MIR, and 15 NIR all placed in one retaining chamber glued to each mouse.

No signs of inflammation were recorded during the trial.

The mice were infested with ticks either 32 days (mouse 1-5 in each group + C6 and C7) or 33 days (mouse 6-10 in each group minus C6 and C7) after immunisation. Each mouse was challenged with one infected *I. ricinus* female + one uninfected *I. ricinus* male and 15 *I. ricinus* nymphs. Ticks fed for 3 days and were then collected. Mice were observed for signs of illness/death for a 21 day period.

### 3.6 Lethal virus challenge

All surviving mice were inoculated intra-peritoneally with approximately 1000 plaque forming units of TBE virus (Hypr strain), 54 days after immunisation, and then monitored for 21 days.

Survivors were bled 20 days after lethal challenge.

### 3.7 Results (Trial 1)

The results are summarised in Table 3 and illustrated in Figure 6.
1. Highest % survival was shown by mice immunised with trp5 or trp2.
2. Transmission-blocking, as measured by the % tick infected, was particularly striking for trp5.
3. The percentage of mice that support virus transmission was lowest for trp5.

### 3.8 Trial II

### Immunisation protocol - Antigens used

TRP-GST fusion proteins including: TRP2, TRP5, and GST protein (control);

### Treatment groups

Two treatments:
1.TRP2 **(A);**
2.TRP5 **(B);**

Two controls:
1.GST protein **(C);**
2.unimmunised **(D).**

**Mouse trial (Balb/c):** 20 mice per treatment group
(A) 15ul TRP2 + 85ul PBS + 100ul TMG = 200ul final volume injected S.C.
(B) 20ul TRP5 + 80ul PBS + 100ul TMG = 200ul final volume injected S.C.
(C) 10ul GST + 90ul PBS + 100ul TMG = 200ul final volume injected S.C.
(D) unimmunised control group
(TMG=TiterMax Gold adjuvant)

### Dates for pre-challenge procedures

1. Immunisations - single dose per mouse were performed on **7/11/01,** (i.e. no boostings were performed)
2. Serum samples - were collected third week post-immunisations **(27-28/11/01)** for serological assays (ELISAs) and mice were challenged at fourth week post-immunisations **(4-7/12/01);**

TBE virus infection via Ixodes ricinus infected adult female ticks co-feeding with uninfected I. r. nymphs was tested for transmission/survival studies (i.e. challenge)

### Results:

The results for survival of mice after commencement of infected tick feeding (in days and date of death) and transmission rate of TBE virus are given below in Table 3:

**Table 3**

| Mouse no. | Survival in D (death date) | Ticks inf./fed (% infected) | Mouse no. | Survival in D (death date) | Ticks inf./fed (% infected) |
|---|---|---|---|---|---|
| A1 | 13(13/12) | 1/11 (9.1%) | B1 | 12 (12/12) | 0/14(0%) |
| A2 | - | ND | B2 | S; **10** (14/01) | 0/3 (0%) |
| A3 | 10 (10/12) | 0/8 (0%) | B3 | S; S | 0/10 (0%) |
| A4 | 18 (18/12) | 0/8 (0%) | B4 | 11 (11/12) | 5/12 (41.7%) |
| A5 | **S;** S | 0/10 (0%) | B5 | S; **10** (14/01) | 0/11 (0%) |
| A6 | 10 (10/12) | 0/ (0%) | B6 | **S**; S | 0/4 (0%) |
| A7 | 10 (10/12) | 2/12 (16.7%) | B7 | 10 (10/12) | 0/7 (0%) |
| A8 | **S;** S | 3/7 (42.9%) | B8 | **S**; S | 2/7 (28.6%) |
| A9 | 10 (10/12) | 1/10 (10%) | B9 | **S**; S | 2/9 (22.2%) |
| A10 | **S;** S | 0/13 (0%) | B10 | 12 (12/12) | 1/10 (10%) |
| **A** | **3/9 (33%)** | **7/87, 8.0%** | **B** | **6/10 (60%)** | **10/87,11.5%** |
| C1 | **S**; S | 3/6 (50%) | D1 | 10 (10/12) | 3/7 (42.8%) |
| C2 | 10 (10/12) | 8/9 (88.9%) | D2 | 12 (12/12) | 7/10 (70%) |
| C3 | 14 (14/12) | 1/11 (9.1%) | D3 | 6 (06/12) | 4/11 (36.4%) |
| C4 | 11 (11/12) | 9/10 (90%) | D4 | 10 (10/12) | 3/8 (37.5%) |
| C5 | 11 (11/12) | 3/10 (30%) | D5 | 10 (10/12) | 1/2 (50%) |
| C6 | 14 (14/12) | 5/7 (71.4%) | D6 | 10 (10/12) | 3/6 (50%) |
| C7 | 13 (13/12) | 5/12 (41.7%) | D7 | 10 (10/12) | 5/11 (45.5%) |
| C8 | 14 (14/12) | 2/9 (22.2%) | D8 | 10 (10/12) | 3/5 (60%) |
| C9 | **S**; S | 4/7 (57.1%) | D9 | 10 (10/12) | 3/4 (75%) |
| C10 | 14 (14/12) | 2/6 (33.3%) | D10 | 10 (10/12) | 4/7(57.1%) |
| **C** | **2/10 (20%)** | **42/87, 48.3%** | **D** | **0/10 (0%)** | **36/71,50.7%** |

| | | | | | |
|---|---|---|---|---|---|
| Notes: ND: not done; **S** - surviving mouse; S - a mouse surviving 2^{nd} challenge; NEG. - donor tick was not infected, a mouse and ticks feeding on that mouse excluded from the trial. | | | | | |

### 2nd challenge control mice - challenge Hypr ip 1000 PFU (04/01/2002):

**1.** D7, **2.** D8, **3.** D8, **4.** D8 - mean incubation period D7.75

A summary of TBE virus transmission in the BALB/c mice is given below in Table 4.

**Table 10**

| **Mouse group & no.** | **Nymphs infected/fed** | **% infected** |
|---|---|---|
| **A1** | 1/11 | 9.1% |
| **A2** | ND | - |
| **A3** | 0/8 | 0% |
| **A4** | 0/8 | 0% |
| **A5** | 0/10 | 0% |
| **A6** | 0/8 | 0% |
| **A7** | 2/12 | 16.7% |
| **A8** | 3/7 | 42.9% |
| **A9** | 1/10 | 10% |
| **A10** | 0/13 | 0% |
| **A** | **7/87** | **8.0% (4/9)** |
| **B1** | 0/14 | 0% |
| **B2** | 0/3 | 0% |
| **B3** | 0/10 | 0% |
| **B4** | 5/12 | 41.7% |
| **B5** | 0/11 | 0% |
| **B6** | 0/4 | 0% |
| **B7** | 0/7 | 0% |
| **B8** | 2/7 | 28.6% |
| **B9** | 2/9 | 22.2% |
| **B10** | 1/10 | 10% |
| **B** | **10/87** | **11.5% (4/10)** |
| **C1** | 3/6 | 50% |
| **C2** | 8/9 | 88.9% |
| **C3** | 1/11 | 9.1% |
| **C4** | 9/10 | 90% |
| **C5** | 3/10 | 30% |
| **C6** | 5/7 | 71.4% |
| **C7** | 5/12 | 41.7% |
| **C8** | 2/9 | 22.2% |
| **C9** | 4/7 | 57.1% |
| **C10** | 2/6 | 33.3% |
| **C=F** | **42/87** | **48.3% (10/10)** |
| **D1** | 3/7 | 42.8% |
| **D2** | 7/10 | 70% |
| **D3** | 4/11 | 36.4% |
| **D4** | 3/8 | 37.5% |
| **D5** | 1/2 | 50% |
| **D6** | 3/6 | 50% |
| **D7** | 5/11 | 45.4% |
| **D8** | 3/5 | 60% |
| **D9** | 3/4 | 75% |
| **D10** | 4/7 | 57.1 |
| **D=H** | **36/71** | **50.7% (10/10)** |

The combined results of TBE virus transmission on TRP immune Balb/c mice, for both Trial I & II are given below in Table 5.

**Table 5**

| Group of mice | Transmission | Support of transmis. | Survival |
|---|---|---|---|
| Group **A** (Trp2) | **14.3%** (22/154) | 59%(10/17) | 41.2% (7/17) |
| Group **B** (Trp5) | **9.4%** (16/180) | 30% (6/20) | 55% (11/20) |
| Group **C** = F (Gst) | **41.9%** (67/160) | 85% (17/20) | 20% (4/20) |
| Group **D** = H | **58.4%** (94/161) | 100% (20/20) | 15% (3/20) |

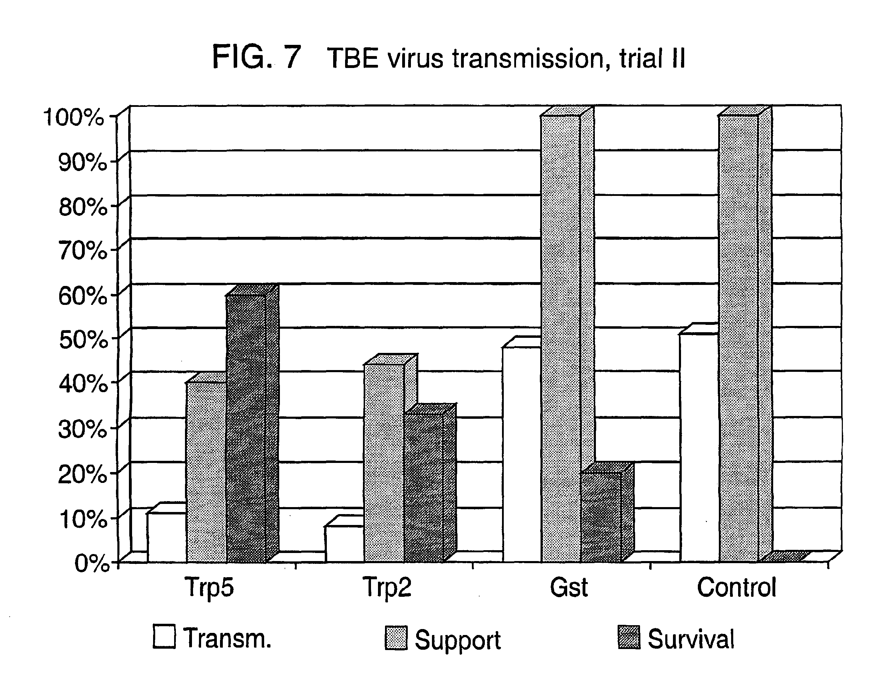

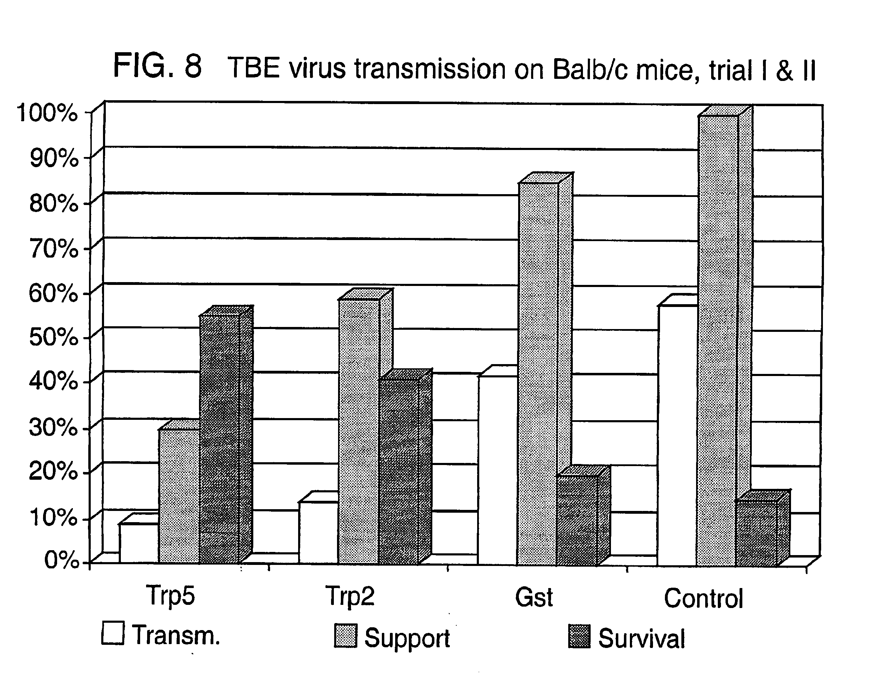

The results of Trial II are illustrated in Figure 7.
The combined results of Trials I and II are illustrated in Figure 8.

### 3.9 Conclusions

Protection against TBEV challenges (i.e. survival of mice) was observed in the TRP2 - and TRP5 -immunised groups (41% and 55%, respectively) compared with much lower survival rate in control groups of unimmunised and GST- immunized mice (15 and 20%, respectively). The infection rate in *I. ricinus* nymphs co-fed on mice immunised with 64TRP5 and 64TRP2 was greatly reduced (14% and 9%, respectively) as compared to nymphs co-fed on unimmunised and GST- immunised control animals (58% and 42%, respectively). Virus propagation (i.e. replication in host cells) was evident in mice from both control groups while only 30% and 59% of 64TRP5 - and 64TRP2 - immunised mice, respectively, supported TBE virus transmission.

Immunisation of Balb/c mice with tick derived recombinant 64TRP2 and 64TRP5 proteins was thus shown to protect the mice against lethal challenge with tick-infected TBE virus, as well as having a virus transmission-blocking effect, suggesting that these proteins provide a considerable degree of protection against virus transmission and against virus-induced death.

### 3.10 Comparison between efficacy of 64TRP constructs and commercial TBE vaccine

Experiments were performed to evaluate the efficacy of a commercial TBE vaccine and 64TRP constructs mentioned herein. The data are presented below in Table 6. These data show that mice immunised with the commercial TBE virus vaccine are protected against lethal challenge with virus-infected ticks (although 2 mice succumbed) but are not protected against infection as shown by their ability to support virus transmission to uninfected ticks feeding upon them.

This result contrasts with the impressive results for 64TRP25 showing markedly reduced levels of infection (and equally impressive protection given that the immunogen is tick derived and unrelated to the virus).

It is possible that the deficiencies of the commercial TBE vaccine can be explained by the fact that development of the commercial TBE vaccine was based on challenging mice with virus inoculated by needle & syringe and NOT delivered by the natural route - an infected tick. It appears that that tick-borne delivery of the virus is able to 'protect' the virus from host immunity to a limited degree that occasionally allows the virus.to 'breakthrough' and cause disease/death.

This result strongly suggests that the efficacy of the constructs mentioned herein in preventing viral transmission might be combined with the advantages of a TBE virus vaccine in protecting against lethal viral challenge with virus-infected ticks. Such a combined vaccine should protect against both infection and death.

**TABLE 6: A summary of transmission efficiency of TBE virus on laboratory mice (+/immunized with 64TRP, or a TBE virus vaccine) and their survival after an infected tick bite; trial I & II**

| Group of mice (immunized with) | Nymphs infected/fed (%) | Mice supporting trans./ used (%)* | Mice survived/used (%) |
|---|---|---|---|
| **A** (TRP2) | 22/144 **(15%)** | 10/16 **(62%)** | 6/16 **(38%)** |
| **B** (TRP5) | 16/173 **(9%)** | 6/19 **(32%)** | 10/19 **(53%)** |
| **F** (GST+TMG) | 67/153 **(44%)** | 17/19 **(90%)** | 3/19 **(16%)** |
| **H** (untreated) | 94/161 **(58%)** | 20/20 **(100%)** | 3/20 **(15%)** |
| **I** (TBE vaccine) | 38/180 **(21%)** | 12/17 **(71%)** | 15/17 **(88%)** |

| | | | |
|---|---|---|---|
| * animals supporting transmission/animals in the experiment; % of animals supporting transmission | | | |

.

## Claims

1. Use of an immunogenic 64p protein consisting of the amino acid sequence presented in Figure 1, an immunogenic fragment thereof or an immunogenic homologue of said 64p protein or fragment having at least 50% identity thereto, or a plasmid DNA vector encoding said protein, fragment or homologue; for the preparation of a vaccine composition for the prevention of tick-borne encephalitis (TBE) and/or to reduce the transmission of the TBE virus to uninfected ticks; wherein said vaccine composition is for administration to animals.

2. Use according to claim 1, wherein said 64p protein, fragment thereof or homologue is expressed in recombinant form.

3. Use according to claim 1 or 2 wherein said vaccine composition additionally comprises a second active agent, wherein said second active agent is an immunogenic protein, the 64p protein, fragments or homologues of the 64p protein, vaccines against infectious diseases and vaccines against TBE.

4. Use according to claim 3 wherein said second active agent is derived from a blood-feeding ectoparasite.

5. Use according to claim 4, wherein said second active agent is an immunogenic 64p protein, an immunogenic fragment thereof or an immunogenic homologue of said protein or fragment having at least 50% identity thereto.

6. Use according to any of the proceeding claims wherein said 64p protein or fragment thereof or homologue is fused to another molecule.

7. Use according to claim 6 wherein said another molecule is a label, a toxin, a bioactive molecule or an immunogenic molecule.

8. Use according to any of the proceeding claims wherein said vaccine composition further comprises an adjuvant.

9. A vaccine composition comprising immunogenic 64p protein consisting of the amino acid sequence presented in Figure 1, an immunogenic fragment thereof or an immunogenic homologue of said 64p protein or fragment having at least 50% identity thereto, or a plasmid DNA vector encoding said protein, fragment or homologue; for use in the prevention of tick-borne encephalitis (TBE) and/or reduction in transmission of the TBE virus to uninfected ticks; wherein said vaccine composition is for administration to animals.

10. The vaccine composition according to claim 9, wherein said 64p protein, fragment thereof or homologue is expressed in recombinant form.

11. The vaccine composition according to claim 9 or 10 wherein said vaccine composition additionally comprises a second active agent, wherein said second active agent is an immunogenic protein, the 64p protein, fragments or homologues of the 64p protein, vaccines against infectious diseases and vaccines against TBE.

12. The vaccine composition according to claim 11 wherein said second active agent is derived from a blood-feeding ectoparasite.

13. The vaccine composition according to claim 12, wherein said second active agent is an immunogenic 64p protein, an immunogenic fragment thereof or an immunogenic homologue of said protein or fragment having at least 50% identity thereto.

14. The vaccine composition according to any of claims 9 to 13 wherein said 64p protein or fragment thereof or homologue is fused to another molecule.

15. The vaccine composition according to claim 14 wherein said another molecule is a label, a toxin, a bioactive molecule or an immunogenic molecule.

16. The vaccine composition according to any claims 9 to 15 wherein said vaccine composition further comprises an adjuvant.

## Patentansprüche

1. Verwendung eines aus der in Figur 1 dargestellten Aminosäuresequenz bestehenden immunogenen 64p-Proteins, eines immunogenen Fragments davon oder eines immunogenen Homologen von diesem 64p-Protein oder von dem Fragment mit wenigstens 50 % Identität damit oder eines Plasmid-DNA-Vektors, der das Protein, Fragment oder Homologe codiert, für die Herstellung einer Impfstoffzusammensetzung zur Vorbeugung von durch Zecken übertragener Enzephalitis (TBE) und/oder um die Übertragung des TBE-Virus auf nicht infizierte Zecken zu reduzieren, wobei die Impfstoffzusammensetzung für die Verabreichung an Tiere ist.

2. Verwendung nach Anspruch 1, wobei das 64p-Protein, das Fragment davon oder das Homologe in rekombinanter Form exprimiert wird.

3. Verwendung nach Anspruch 1 oder 2, wobei die Impfstoffzusammensetzung zusätzlich ein zweites aktives Mittel umfasst, wobei das zweite aktive Mittel ein immunogenes Protein, das 64p-Protein, Fragmente oder Homologe des 64p-Proteins, Impfstoffe gegen Infektionskrankheiten oder Impfstoffe gegen TBE sind.

4. Verwendung nach Anspruch 3, wobei das zweite aktive Mittel aus einem sich von Blut ernährenden Ektoparasiten ist.

5. Verwendung nach Anspruch 4, wobei das zweite aktive Mittel ein immunogenes 64p-Protein, ein immunogenes Fragment davon oder ein immunogenes Homologes des Proteins oder Fragments mit wenigstens 50 % Identität damit ist.

6. Verwendung nach einem der vorangehenden Ansprüche, wobei das 64p-Protein oder das Fragment davon oder das Homologe mit einem anderen Molekül fusioniert ist.

7. Verwendung nach Anspruch 6, wobei das andere Molekül eine Markierung, ein Toxin, ein bioaktives Molekül oder ein immunogenes Molekül ist.

8. Verwendung nach einem der vorangehenden Ansprüche, wobei die Impfstoffzusammensetzung weiterhin ein Adjuvanz umfasst.

9. Impfstoffzusammensetzung, umfassend ein aus der in Figur 1 dargestellten Aminosäuresequenz bestehendes immunogenes 64p-Protein, ein immunogenes Fragment davon oder ein immunogenes Homologes des 64p-Proteins oder des Fragments mit wenigstens 50 % Identität damit oder ein Plasmid-DNA-Vektor, der das Protein, Fragment oder Homologe codiert für die Verwendung bei der Vorbeugung von durch Zecken übertragener Enzephalitis (TBE) und/oder für die Reduzierung der Übertragung des TBE-Virus auf nicht infizierte Zecken, wobei die Impfstoffzusammensetzung für die Verabreichung an Tiere ist.

10. Impfstoffzusammensetzung nach Anspruch 9, wobei das 64p-Protein, das Fragment davon oder das Homologe in rekombinanter Form exprimiert wird.

11. Impfstoffzusammensetzung nach Anspruch 9 oder 10, wobei die Impfstoffzusammensetzung zusätzlich ein zweites aktives Mittel umfasst, wobei das zweite aktive Mittel ein immunogenes Protein, das 64p-Protein, Fragmente oder Homologe des 64p-Proteins, Impfstoffe gegen Infektionskrankheiten oder Impfstoffe gegen TBE sind.

12. Impfstoffzusammensetzung nach Anspruch 11, wobei das zweite aktive Mittel aus einem sich von Blut ernährenden Ektoparasiten ist.

13. Impfstoffzusammensetzung nach Anspruch 12, wobei das zweite aktive Mittel ein immunogenes 64p-Protein, ein immunogenes Fragment davon oder ein immunogenes Homologes des Proteins oder Fragments mit wenigstens 50 % Identität damit ist.

14. Impfstoffzusammensetzung nach einem der Ansprüche 9 bis 13, wobei das 64p-Protein oder das Fragment davon oder das Homologe mit einem anderen Molekül fusioniert ist.

15. Impfstoffzusammensetzung nach Anspruch 14, wobei das andere Molekül eine Markierung, ein Toxin, ein bioaktives Molekül oder ein immunogenes Molekül ist.

16. Impfstoffzusammensetzung nach einem der Ansprüche 9 bis 15, wobei die Impfstoffzusammensetzung weiterhin ein Adjuvanz umfasst.

## Revendications

1. Utilisation d'une protéine 64p immunogène constituée de la séquence d'acides aminés présentée sur la figure 1, d'un de ses fragments immunogènes ou d'un homologue immunogène de ladite protéine 64p ou dudit fragment ayant au moins 50 % d'identité à ceux-ci ou un vecteur d'ADN plasmide codant ladite protéine, le fragment ou l'homologue ; pour la préparation d'une composition de vaccin pour la prévention de l'encéphalite à tiques (TBE) et/ou visant à réduire la transmission du virus de TBE aux tiques non infectés ; ladite composition de vaccin étant destinée à une administration aux animaux.

2. Utilisation selon la revendication 1, dans laquelle ladite protéine 64p, son fragment ou son homologue, est exprimé(e) sous une forme recombinante.

3. Utilisation selon la revendication 1 ou la revendication 2, dans laquelle ladite composition de vaccin comprend en outre un second agent actif, où ledit second agent actif est une protéine immunogène, la protéine 64p, des fragments ou homologues de la protéine 64p, des vaccins contre les maladies infectieuses et des vaccins contre la TBE.

4. Utilisation selon la revendication 3, dans laquelle ledit second agent actif est dérivé d'un ectoparasite se nourrissant de sang.

5. Utilisation selon la revendication 4, dans laquelle ledit second agent actif est une protéine 64p immunogène, un de ses fragments immunogènes ou un homologue immunogène de ladite protéine ou dudit fragment ayant au moins 50 % d'identité avec ceux-ci.

6. Utilisation selon l'une quelconque des revendications précédentes, dans laquelle ladite protéine 64p ou son fragment ou homologue est fusionné(e) à une autre molécule.

7. Utilisation selon la revendication 6, dans laquelle ladite autre molécule est un marqueur, une toxine, une molécule bioactive ou une molécule immunogène.

8. Utilisation selon l'une quelconque des revendications précédentes, dans laquelle ladite composition de vaccin comprend en outre un adjuvant.

9. Composition de vaccin comprenant une protéine 64p immunogène, constituée de la séquence d'acides aminés présentée sur la figure 1, un de ses fragments immunogènes ou un homologue immunogène de ladite protéine 64p ou dudit fragment ayant au moins 50 % d'identité avec ceux-ci, ou un vecteur d'ADN plasmide codant ladite protéine, ledit fragment ou homologue ; pour une utilisation dans la prévention de l'encéphalite à tiques (TBE) et/ou visant à réduire la transmission du virus de la TBE aux tiques non infectées ; ladite composition de vaccin étant destinée à une administration aux animaux.

10. Composition de vaccin selon la revendication 9, dans laquelle ladite protéine 64p, son fragment ou homologue, est exprimé(e) sous une forme recombinante.

11. Composition de vaccin selon la revendication 9 ou la revendication 10, dans laquelle ladite composition de vaccin comprend en outre un second agent actif, où ledit second agent actif est une protéine immunogène, la protéine 64p, des fragments ou homologues de la protéine 64p, des vaccins contre les maladies infectieuses et des vaccins contre la TBE.

12. Composition de vaccin selon la revendication 11, dans laquelle ledit second agent actif est tiré d'un ectoparasite se nourrissant de sang.

13. Composition de vaccin selon la revendication 12, dans laquelle ledit second agent actif est une protéine 64p immunogène, un de ses fragments immunogènes ou un homologue immunogène de ladite protéine ou dudit fragment ayant au moins 50 % d'identité avec ceux-ci.

14. Composition de vaccin selon l'une quelconque des revendications 9 à 13, dans laquelle ladite protéine 64p ou son fragment ou homologue est fusionné(e) à une autre molécule.

15. Composition de vaccin selon la revendication 14, dans laquelle ladite autre molécule est un marqueur, une toxine, une molécule bioactive ou une molécule immunogène.

16. Composition de vaccin selon l'une quelconque des revendications 9 à 15, dans laquelle ladite composition de vaccin comprend en outre un adjuvant.
